(19) Europäisches Patentamt
European Patent Office
Office européen des brevets

(11) EP 1 435 929 B1

(12) **EUROPEAN PATENT SPECIFICATION**

(45) Date of publication and mention of the grant of the patent:
**16.01.2008 Bulletin 2008/03**

(21) Application number: **02798740.3**

(22) Date of filing: **18.09.2002**

(51) Int Cl.:
***A61K 31/13*** *(2006.01)* ***A61P 15/14*** *(2006.01)*
***A61K 47/40*** *(2006.01)*

(86) International application number:
**PCT/EP2002/010518**

(87) International publication number:
**WO 2003/024438 (27.03.2003 Gazette 2003/13)**

(54) **COMPOSITION COMPRISING CYSTEAMINE FOR IMPROVING LACTATION IN DAIRY ANIMALS**

ZUSAMMENSETZUNG MIT CYSTEAMIN ZUR VERBESSERUNG DER LAKTATION VON MILCHVIEH

COMPOSITION CONTENANT DE LA CYSTEAMINE DESTINEE A AMELIORER LA LACTATION DES ANIMAUX PRODUCTEURS DE LAIT

(84) Designated Contracting States:
**AT BE BG CH CY CZ DE DK EE ES FI FR GR IE IT LI LU MC NL PT SE SK TR**

(30) Priority: **19.09.2001 GB 0122610**

(43) Date of publication of application:
**14.07.2004 Bulletin 2004/29**

(73) Proprietor: **Walcom Animal Science (I.P.) Limited**
**Kowloon, Hong Kong (CN)**

(72) Inventors:
- **CHI, Francis**
**Tsimshatsui,**
**Kowloon,**
**Hong Kong (CN)**
- **WEN, Qin, Tang**
**Tsimshatsui,**
**Kowloon,**
**Hong Kong (CN)**
- **LU, Tian, Shui**
**Tsimshatsui, Kowloon, Hong Kong (CN)**

(74) Representative: **Arends, William Gerrit et al**
**Marks & Clerk**
**90 Long Acre**
**London WC2E 9RA (GB)**

(56) References cited:

| | |
|---|---|
| **WO-A-02/48110** | **WO-A-96/12493** |
| **WO-A-97/18245** | **WO-A-97/35568** |
| **WO-A-98/40069** | **FR-A- 2 716 625** |
| **JP-A- 11 246 603** | **US-A- 4 711 897** |

- **ROTT F.: "Neue Erkenntnisse über den Wirkungsmechanismus von Cysteamin in der Therapie der Azetonämie und Laktationsverbesserung." TIERAERZTLICHE UMSCHAU, vol. 43, no. 5, 1988, pages 324-326, XP008012507**
- **WANG YAN-LING ET AL: "Effects of cysteamine on milk yield and several hormones levels of blood in rats at late stage of lactation." ZOOLOGICAL RESEARCH, vol. 19, no. 3, 1998, pages 247-249, XP008012494 ISSN: 0254-5853**
- **MILLARD, W.J., ET AL.: "Effect of cysteamine on suckling-indued prolactine secretion in the rat." LIFE SCIENCES, vol. 41, no. 20, 1987, pages 2255-2260, XP008012495**
- **BOULET Y ET AL: "CHARACTERIZATION OF CYSTEAMINE COMPLEXES IN ALPHA, BETA AND GAMMA-CYCLODEXTRINS" PROCEEDINGS OF THE INTERNATIONAL SYMPOSIUM ON CYCLODEXTRINS, XX, XX, 21 May 2000 (2000-05-21), pages 1-5, XP008012502**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 435 929 B1

## Description

Background of Invention

1. Field of Invention

The present invention relates to the uses of cysteamine or a cysteamine-containing composition for improving lactation of lactating animals, and in particular, but not limited to, dairy animals such as dairy cows. The present invention also relates to a method for improving lactation of lactating animals.

2. Description of Prior Art

Studies have indicated that growth hormones play an important role in regulating lactation in dairy animals such as diary cows. Further studies on dairy animals have shown that milk production therefrom can be increased when exogenous growth hormones are administered to the animals. However, there are a number of disadvantages in directly using growth hormones in increasing milk production. Firstly, growth hormones from different animals are seldom homogenous and different dairy animals only react to certain types of specific growth hormones. Since suitable exogenous growth hormones are normally extracted from pituitary glands, it is rather difficult and uneconomical to prepare sufficient quantity of suitable exogenous growth hormones for use on a large-scale application. Although exogenous growth hormones can now be prepared using DNA recombinant technology, exogenous growth hormones manufactured by such method are still rather expensive. Secondly, the administration of exogenous growth hormones into dairy animals is normally performed by direct injection, which is inevitably rather costly and difficult to administer in a large farm. Thirdly, it is rather difficult to control the dose administered to produce precisely the desired effect, and an overdose of exogenous growth hormones is likely to be harmful to the animals. Fourthly, residuals of these exogenous growth hormones may be passed to the dairy products and subsequently to humans through consumption thereof. Further studies in this regard are required although some scientists are concerned about the negative side effects of these exogenous growth hormones to humans.

Cysteamine is a component of co-enzyme A and works as a physiological regulator. Cysteamine has been used as an additive in feed in promoting growth of meat-producing animals. US Patent No. 4,711,897 discloses animal feed methods and feed compositions comprising cysteamine.

However, it has been identified that cysteamine is a fairly sensitive and unstable compound under normal room temperature conditions. For example, cysteamine is readily oxidized when exposed to air or at an elevated temperature. Cysteamine is highly hydroscopic. Also, cysteamine is unpalatable when taken directly by mouth. Further, ingesting cysteamine directly will cause undesirable gastro side effects. For these reasons, the use of cysteamine had for a long time been limited to direct injection of cysteamine-containing solution into meat-producing animals.

PRC Patent Publication No. CN1358499 and International Publication No. WO/0248110 disclose an improvement of a cysteamine-containing composition which can be mixed with basal animal feed to promote growth and increase in animal weight. However, there continues to exist a need for a composition, feed and/or method for increasing lactation by dairy mammals and particularly dairy cows. Preferably, the composition and method are safe and can be easily administered and inexpensive to carry out.

Tierärztl. Umschau 43, 324-328 (1988) by von F. Rott discloses new findings on the mechanism of action of cysteamine in the therapy of ketosis and lactation improvement.

Zoological Research 1998, 19(3): 247-249 by Wang et al discloses the effect of cysteamine on milk yield and several hormone levels in blood of rats at late stage of lactation.

It is thus an object of the present invention in which the above issues are addressed, or at least to provide a useful alternative to the public.

Summary of Invention

According to a first aspect of the present invention, there is provided the use of a lactation increasing composition for improving lactation of lactating animals, wherein said composition comprises (i) 1 to 95wt% cysteamine or a salt thereof, (ii) an inclusion compound host material stabilizer for protecting said cysteamine or a salt thereof in said composition from light, heat, air and moisture of the surroundings, and (iii) a coated carrier, and wherein the use is non-therapeutic.

According to a second aspect of the present invention, there is provided the use of a composition comprises an inclusion compound host material stabilizer for protecting said cysteamine or a salt thereof from light, heat, air and moisture of the surroundings in said composition, and further comprises a coated carrier for the manufacture of lactation improving medicament for lactating animals.

According to a third aspect of the present invention, there is provided a method for improving lactation of lactating animals comprising:

(c) producing a final feed by mixing a composition containing (i) cysteamine or a salt thereof, (ii) an inclusion compound host material stabilizer for protecting said cysteamine or salt thereof from light, heat, air and moisture of the surrounding, and (iii) a coated carrier with a suitable basal feed for said animals; and
(d) feeding said animals with said final feed;
wherein said method is non-therapeutic.

Also disclosed herein a feed for improving lactation of lactating animals comprising a composition containing cysteamine or a salt thereof and a stabilizer.

The improving lactation may be an increase in milk yields, fat-corrected milk yields, milk fat content therein and/or milk protein content therein.

Preferably, the composition comprises 1 to 95wt% cysteamine having the chemical formula of $NH_2$-$CH_2$-$CH_2$-SH, or a salt thereof. In particular, the composition may comprise 30wt% cysteamine.

Suitably, the composition comprises 1 to 80wt% of the stabilizer. The stabilizer is preferably selected from a group including cyclodextrin or a derivative thereof. In particular, the composition may comprise 10wt% of the stabilizer.

Advantageously, the composition further comprises ingredient(s) selected from a group including a bulking agent, and a disintegration agent. Preferably, the coated carrier is a solid carrier which is a coating soluble in intestines of the animals. The coated carrier suitably exhibits a multi-layer structure in the composition. The coated carrier is preferably adapted to remain un-dissolved at pH 1.5 to 3.5.

Preferably, the feed comprises 400 to 1000ppm of the composition, or 1000 to 2174ppm of the composition when in its dry state. Alternatively, the feed comprises 120 to 300ppm cysteamine, or 200 to 650ppm cysteamine when in its dry state.

In some embodiments, the feed comprises other foodstuffs selected from a group including normal premix, cornmeal, cotton seed, wheat gluten, maize silage rutabaga, sugar beet pulp, apple pulp, ryegrass, fescue grass, alfalfa, feed concentrate and feed supplement.

In one embodiment, the mixing comprises directly mixing the composition with the basal feed. In another embodiment, the mixing comprises firstly preparing a premix including cysteamine or the composition, and subsequently mixing the pre-mix with the basal feed. The premix is prepared by mixing cysteamine or the composition with a food material such as cornmeal. The premix preferably comprises 5 to 25wt% of the composition. In particular, the premix may comprise 10 to 20wt% of the composition.

Preferably, the animals are fed with 5.64 to 12.71g cysteamine, or a salt thereof, or 19.79 to 42.36g of the composition, per animal per day. Alternatively, the animals are fed with twice the amount of cysteamine or the composition but the frequency of the administration thereof can be reduced to every other day instead of every day. Similar results would be produced.
The lactating animals referred above may be dairy cows.

## Brief Description of Drawing

The invention will now be described, by way of non-limiting examples only, with reference to the accompanying drawing, in which:-

Fig. 1 is a graph showing level of lactation of three groups of dairy cows in an experiment.

## Detailed Description of the Invention

The present invention is based on the demonstration that cysteamine or a cysteamine-containing composition when administered to lactating animals such as diary cows has activity in improving lactation therefrom. The lactating animals or dairy animals referred hereinafter include any milk-producing animals such as dairy cows. Prior to this finding, there was no suggestion or sufficient indication that cysteamine or its variants or derivatives might have such activity. The present invention also provides a method for improving lactation of lactating animal. The use of the present invention also prolongs and heightens the milk yield during later stage of lactation of dairy animals. The invention may be practiced by directly mixing cysteamine or the cysteamine-containing composition with a suitable basal feed. Alternatively, the invention may be practiced by mixing firstly a premix made of cysteamine or the cysteamine-containing composition and other ingredients such as cornmeal, and secondly the premix with a suitable basal feed to form a final feed.

One effect of cysteamine is described in PRC Patent Publication No. CN1358499, the content of which is incorporated herein. One effect of cysteamine is its effect on improving lactation of lactating animals. The effect of

cysteamine or a cysteamine-containing composition on lactating animals is explained as follows. It is believed that cysteamine having a physiological activity acts as a growth stimulator. Natural cysteamine is a part of coenzyme A (also know as CoA-SH or CoA) which is a coenzyme pattern of pantothenic acid. In the course of metabolism, coenzyme A acts as the carrier of dihydrosulfuryl or variants of hydrosulfuryl which is linked with the hydrosulfuryl of coenzyme A. Experiments performed on other animals such as pigs, poultry, fowls, goats, rabbits and fish have shown that cysteamine can deplete somatostain (SS) in the animals. This increases the level of growth hormone in the blood of the animals which at the same time raises the level of various other growth stimulating factors including insulin-like growth factor I (IGF-I), insulin, triiodothyronine (T3), trthyroxine (T4) and beta-endorphin (beta-END). As regards the present invention, the growth hormone is believed to directly stimulate the development and activity of mammalian glands of dairy animals and the maintenance of lactation therefrom.

With the increase of these various growth-promoting factors, the digestive metabolic rate of the animals is correspondingly increased. It is understood that the general protein synthesis rate of the animals is accordingly increased, and thus lactation is increased.

The cysteamine-containing composition used in embodiments of the present invention comprises two main ingredients of 1 to 95wt% of cysteamine (or its salts, for example, cysteamine hydrochloride, or other pharmaceutically acceptable acid addition salts thereof) and 1 to 80wt% of a carrier which is an inclusion compound host material. The chemical formula of cysteamine is $HSCH_2CH_2NH_2$. The term "cysteamine" referred hereinafter means cysteamine and/or its salt like compounds. Cysteamine and its salts are well known in the chemical literature. The general chemical formula of a cysteamine salt is $C_2H_7NS$ . X, where X may be HCl, $H_3PO_4$, bitartrate, salicylate, etc. The cysteamine used is preferably of pharmaceutically acceptable standard and the content of carbon, hydrogen, nitrogen and sulfur therein are 31.14wt%, 9.15wt%, 18.16wt% and 41.56wt% respectively. While the workable content of cysteamine in the cysteamine-containing composition ranges from 1 to 95wt%, a preferable range of 1 to 75wt% and a more preferable range of 1 to 40wt% of cysteamine may be used. Cysteamine is one of the main active ingredients of the cysteamine-containing composition. However, it has been identified that if the content of cysteamine in the cysteamine-containing composition exceeds 95wt%, mixing the composition with a basal feed would be rather difficult and the effect of the composition for regulating lactation of the dairy animals would be hindered.

The inclusion compound host material may comprise mainly cyclodextrin and/or its derivatives which are selected from a group including methyl β-cyclodextrin (M-β-CD) , hydropropyl β-cyclodextrin (HP-β-CD) , hydroethyl β-cyclodextrin (HE-β-CD), polycyclodextrin, ethyl β-cyclodextrin (E-β-CD) and branched cyclodextrin. The general chemical formula of cyclodextrin is $(C_6O_5H_9)_n \cdot (C_6O_5H_9)_2$ and the structural formula is as follows.

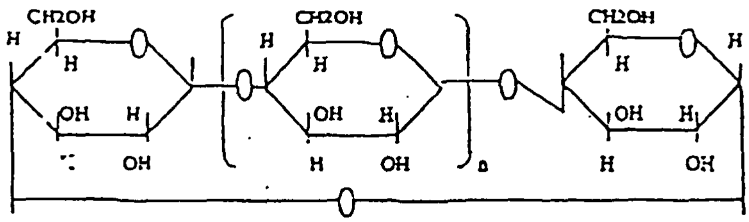

where α-CD n=4; β-CD n=5; γ-CD n=6.
(Cyclodextrin is a cyclic oligomer of alpha-D-glucopyranose.)

It is worthwhile to note that the β-CD form of cyclodextrin is preferably used because the internal diameter of its molecule is about 6-8Å which makes it a particular suitable candidate as an inclusion compound host material for preparation of the cysteamine-containing composition, which involves the use of an inclusion process. The term "cyclodextrin" referred hereinafter means cyclodextrin and/or its derivatives. Any derivative of cyclodextrin which has the property of stabilizing and protecting cysteamine from degradation may be used. For example, any one of the group of cyclodextrin or its derivatives mentioned above may be used.

While the workable content of the inclusion compound host materials in the cysteamine-containing composition ranges from 1 to 80wt%, a preferable workable range of 1 to 60wt% and a more preferable workable range of 10 to 40wt% of the inclusion compound host materials may be also be used. The actual amount of the inclusion compound host materials used will depend on the actual content of the cysteamine used in preparing the cysteamine-containing composition.

The cysteamine-containing composition may also comprise 1 to 90wt% of fillers although a preferable workable range of 1 to 60wt% and a more preferable workable range of 1 to 40wt% of the fillers may also be used in the composition. The actual content will depend on the actual amount of cysteamine and inclusion compound host materials used. The fillers may be selected from a group including powdered cellulose, starch and calcium sulfate (e.g. $CaSO_4.2H_2O$). It is

to be noted that if the content of the fillers exceeds 90wt% in the cysteamine-containing composition, the content of the main active ingredients will thus be reduced, and the cysteamine-containing composition may become ineffective in improving lactation of the animals fed with a feed mixed therewith.

The cysteamine-containing composition may also comprise 5 to 50wt% of disintegrants and binders although a preferable workable range of 10 to 40wt% and a more preferable workable range of 15 to 35wt% may also be used. The actual content will depend on the actual amount of cysteamine, the inclusion compound host material and other ingredients used. The binders and disintegrants may be selected from a group including hydropropyl starch, microbial alginate, microcrystalline cellulose and starch. It has been identified that if the content of the disintegrants and binders in the composition is less than 5wt%, granules of the composition produced will lack the required hardness. In addition, manufacturing of the composition would become very difficult. If however the content of the disintegrants and binders is more than 50wt%, the resulting composition will have excessive hardness, this is especially so if the content of binders represent a large portion of the mixture of the disintegrants and binders. This will result in difficult absorption of the composition by the intestines of the animals.

The cysteamine-containing composition may also comprise 0.05 to 0.3wt% of flavoring and smelling agents which may be a flavoring essence.

The cysteamine-containing composition comprises a coated carrier and may comprise 1 to 20wt% of coating materials although a preferable workable range is 1 to 15wt% and a more preferable workable range is 2 to 10wt%. The actual content will depend on the actual amount of cysteamine, the inclusion compound host materials and the other ingredients used. The coating materials are preferably enteric-coated which allows dissolution in an alkaline environment such as in the intestines. The coating materials may be made of and selected from a group including cellulose acetate phthalate, starch acetate phthalate, methyl cellulose phthalate, glucose or fructose derivatives from phthalic acid, acrylic and methacrylic copolymers, polymethyl vinyl ether, partly esterified substance of maleic anhydride copolymers, lac and formogelatine. It has been identified that if the content of the coating materials is less than 1wt%, granules of the composition may not be entirely covered by the coating materials which act as a protective layer. The cysteamine-containing composition may thus degrade before being absorbed by the intestines into the bloodstream of the animals. On the other hand, if the content of the coating materials exceeds 15wt%, the active ingredients in the composition may not effectively be released from the composition. Thus, the intended regulation of lactation would not be achieved.

The cysteamine-containing composition used in the present invention is in the form of small granules each of which has a preferable diameter of 0.28 to 0.90mm. These granules are prepared using a micro-encapsulation method. The method involves using a macromolecular substance having inclusion property, which is the inclusion compound host material (which may comprise mainly cyclodextrin) described above. The inclusion compound host material is a macromolecular substance which acts as a molecular capsule to engulf the molecules of cysteamine, whereby cysteamine in the composition is protected and insulated from light, heat, air and moisture of the surroundings. The stability of cysteamine is thus preserved. The inclusion compound host material used in the micro-encapsulation method is preferably a cyclic polysaccharide compound having 6 to 12 glucose molecules, which is produced by reacting cyclodextrin glycosidtransferase and starch in the presence of *Bacillus.* Various studies using acute, sub-acute and chronic toxic tests have shown that the macromolecular substance is non-toxic. Subsequent to the micro-encapsulation process, each granule is coated with at least one and preferably a plurality of layers of the coating materials described above. The following provides a more detailed description of a method of preparing the cysteamine-containing composition used in the present invention.

In a jacketed reactor linked with polytetrafluoroethylene and equipped with a polytetrafluoroethylene coated stirrer, 4080g of 75wt% cysteamine hydrochloride solution in ethanol is added with mainly nitrogen being the atmosphere. The purity, melting point and burning residue of the cysteamine used are preferably 98% or above, 66 to 70°C and 0.05% or below respectively. 1200g β-cyclodextrin is then added into the reactor similarly under the protection of nitrogen gas. (The quality of β-cyclodextrin is in accordance with the requirements for a food additive. In particular, the dry basis purity is more than 98%; the weight loss by drying is less than 10.0%; the burning residue is less than 0.2%; the content of heavy metal is less than 10ppm; the arsenic content is less than 2ppm.) The mixture is then heated for 3 hours at 40°C. Heating is then stopped and stirring continues for two hours thereafter, products resulted therefrom are then grounded and sieved through a screen (e.g. 40-mesh) filter after the products have been vacuum dried at a temperature of 40-50°C. All parts of the equipment, which may come in contact with the ingredients of the composition, should preferably be made of stainless steel.

In a tank-type mixer, 4200g (on dry basis) of the cysteamine which has undergone the inclusion process as described, 2600g of the fillers, and 1200g of the disintegrants and 1700g binders are added under the protection of a dry surroundings. These ingredients are then thoroughly mixed, and a suitable amount of anhydrous ethanol may be added and then mixed therewith. The resulting mixture presents a soft material with moderate hardness, so that it can be shaped into a ball by a light hold of palms. The ball-shaped resulting mixture may then be broken up by a light touch. After the mixture is pelleted by a granulator under the protection of nitrogen, the small granules resulting therefrom is immediately introduced to a fluid-bed dryer, and is then dried at the temperature of 40-50°C in a substantially vacuum

environment.

Enteric coating materials are then prepared by a method with the following formulation: cellulose acetate phthalate 8.0g, polyethylene glycol terephthalate 2.4 ml, ethyl acetate 33.0ml and isopropyl acetate 33.6 ml. The resultant granules obtained above are uniformly coated under the protection of nitrogen with at least one layer but preferably a plurality of layers of the enteric coating materials described above. The enteric coating materials are dissolvable only at an alkaline environment. This can prevent the cysteamine from prematurely escaped from the composition while it is still in the stomach of the animal. Cysteamine can adversely stimulate gastric mucous of the stomach of the animals.

The resultant granules of the cysteamine-containing composition are then dried completely in a substantially vacuum dryer at a temperature of 40 to 50°C. Then, all solvents are removed. The resultant granules are then allowed to cool to room temperature, the micro-capsules were mixed with a suitable amount of flavoring and smelling agents by a cantilever double helix blender. The cysteamine-containing composition is a microcapsule with its interior having cysteamine hydrochloride and cyclodextrin, and with its exterior coated with the enteric coating materials.

The composition produced will exhibit small granular (or micro-particulate) shape having smooth surface, good flow property, and is easy to be blended with various animal feeds. The diameter of each granule of the composition is preferably 0.28 to 0.90mm. The composition also has excellent stability. It has been found that after the composition is packaged with sealed plastic bags and stored for one year in a cool, dark and dry place, their properties remain unchanged. Therefore, they meet the requirements for a feed additive.

The composition having the particular construction described above has a number of functional advantages over cysteamine by itself. Firstly, the activity of the cysteamine contained in the composition is preserved after it has been produced. This is important as feed additive such as the composition may be stored for a relatively long period of time before use. Secondly, the composition does not cause any noticeable gastro side effects to the animals fed therewith. Thirdly, the activity of the composition is preserved not only during storage but more importantly until it reaches the intestines of the animals. Fourthly, the composition can be easily administered to farm animals on a large scale basis cost-effectively because it can be readily mixed with any basal feed. No separate procedure or injection is needed at all.

Various experiments have been conducted to demonstrate that administering a feed having cysteamine or a cysteamine-containing composition increases lactation from dairy animals, three experiments of which are described in detail as follows.

<u>EXPERIMENTS</u>

<u>Experiment 1</u>

Background Information

The experiment was conducted in a private dairy farm located in Guangming, PRC in April and May 2001. Thirty dairy cows were randomly selected for the experiment. Before the experiment, the cows were similar in weight, age, milk yield, and milk fat content and have similar calving number. All the cows had had approximately five months of lactation. The cows were randomly divided into three groups of ten, namely Group I, Group II and Group III, Group III being in the control. Prior to the experiment, there was no statistical difference ($p>0.05$) on average milk yield among the three groups of cows. Table 1 below shows data in respect of the three groups of cows and milk produced therefrom prior to the experiment.

**Table 1:** Characteristics of diary cows before experiment

| Characteristics/Group | Group I | Group II | Group III |
|---|---|---|---|
| **Number of cows** | 10 | 10 | 10 |
| **Average number of calving** | 1 | 1 | 1 |
| **Average lactation period (days)** | 212.8 ± 5.20 | 208.4 ± 4.05 | 208.3 ± 3.91 |
| **Average milk yield (kg/day)** | 27.5 ± 4.27 | 27.4 ± 4.05 | 27.5 ± 3.97 |
| **FCM (3.5%, kg/day)** | 28.636 ± 5.246 | 28.773 ± 5.777 | 28.978 ± 4.973 |
| **Average fat content in FCM (wt%)** | 3.76 ± 0.41 | 3.78 ± 0.37 | 3.84 ± 0.39 |
| **Average protein content in milk (g/day)** | 856.6 ± 109.1 | 964.7 ± 159.6 | 869.8 ± 112.7 |

(continued)

| Characteristics/Group | Group I | Group II | Group III |
|---|---|---|---|
| **Average number of somatic cells in milk (x 1000/ml)** | 91.7 ± 58.7 | 86.3 ± 49.99 | 84.9 ± 58.5 |
| Note: FCM = fat corrected milk | | | |

It is to be noted that during a lactation period, the content of milk proteins and other nutrients in milk may often remain relatively stable, whereas the fat content can vary substantially. In this regard, it has become one of the international standards to express the quantity of milk produced in terms of its equivalent quantity of fat corrected milk (FCM) for unified comparison. In the experiment, the quantity of milk produced by the three groups of dairy cows are expressed both in its original quantity (see Table 7) as well as quantity in 3.5wt% FCM (see Table 8). It is however to be noted that there are other standards for quantifying milk production.

Materials

The recipe of two basal feeds used in the experiment are shown in Table 2 below, and the details of the respective diets of the three groups of cows are described below.

**Table 2**: Composition of basal feeds

| Ingredients (kg) | For use before April 30, 2001 | For use on or after April 30, 2001 |
|---|---|---|
| **Basal concentrate** | 5 | 4.5 |
| **Supplement feed** | 3 | 3 |
| **Normal premix** | 1.4 | 1.3 |
| **Cotton seed** | 0.4 | 0.2 |
| **Wheat gluten** | 10 | 11 |
| **Maize silage** | 14 | 14.5 |
| **Rutabaga** | 4 | 5 |
| **Sugar beet pulp** | 0.5 | 0.4 |
| **Apple pulp** | 0.5 | 0.4 |
| **Ryegrass** | 4 | - |
| **Fescue grass** | 3 | 3 |
| **Alfalfa** | 0.8 | 0.5 |
| **Total (Basal feed)** | 46.6 | 43.8 |

Two batches of the basal feeds were prepared, the first batch was used in the first period of the experiment, i.e. from 11 April to 29 April 2001 while the second batch was used in the second period of the experiment, i.e. from 30 April to 29 May 2001. The different basal feed was used in the second period of the experiment in order to suit the physiological requirements of the dairy cows at that particular developmental stage. As shown in Table 2 above, the first batch of feed comprised a plurality of ingredients including basal concentrate, feed supplement, normal premix, cottonseed, wheat gluten, maize silage, rutabaga, sugar beet pulp, apple pulp, ryegrass, fescue grass and alfalfa. The normal premix is formulated to compose ingredients such as vitamins and minerals, which are suitable for the cows in a particular physiological stage. The basal concentrate, supplement feed and normal premix were nutrition additives containing such as vitamins and rare elements, which were added in the feed in accordance with America's Animal National Nutrition standard. The second batch of basal feed generally comprised the same ingredients although the quantities thereof were different.

The basal feeds were used to prepare final feeds. Referring to Table 3 below, the final feeds comprised 200g of a specific premix which was prepared by mixing a cysteamine-containing composition with a suitable food material such as cornmeal. It is to be noted that the specific premix was different from the normal premix in the basal feed.

**Table 3:** Composition of final feeds for Groups I and II cows

| Ingredients (kg) | For use before April 30,2001 | For use on or after April 30, 2001 |
|---|---|---|
| **Specific premix** | 0.2 | 0.2 |
| **Basal feed** | 46.6 | 43.8 |
| **Total (final feed)** | 46.8 | 44.0 |

There were however three formulas of the specific premix, each prepared with different amounts of the cysteamine-containing composition and the food material. These formulas are illustrated as follows.

Formula 1: 10wt% cysteamine-containing composition/90wt% cornmeal
Formula 2: 20wt% cysteamine-containing composition/80wt% cornmeal
Formula 3: 0wt% cysteamine-containing composition/100wt% cornmeal

While formulas 1 to 3 of the specific premix comprises up to 20wt% of the cysteamine-containing composition, studies have shown that, in practice, the specific premix may have a content of 5 to 25wt% of the cysteamine-containing composition and a similar effect on increasing lactation of the dairy cows will result as illustrated below.

Tables 4 to 6 below further summarize the respective diets of the Groups I to III cows during the experiment. Based on the data in Table 4, it is calculated that the concentrations of the cysteamine-containing composition in the final feeds (or diets) of the Group I cows in the first and second periods of the experiment were 427 and 455ppm respectively. Based on the data in Table 5, it is calculated that the concentrations of the cysteamine-containing composition in the final feeds (or diets) of the Group II cows in the first and second periods were 855 and 909ppm respectively. Referring to Table 6, no cysteamine-containing composition was added in preparing the 180g of specific premix (Formula III). Therefore, the final feeds (or diets) of the Group III cows did not contain any cysteamine-containing composition.

**Table 4:** Diets of the Group I dairy cows

| Ingredients | For Use Before April 30 | For Use On or After April 30 |
|---|---|---|
| **Basal feed (g)** | 46600 | 43800 |
| **Specific premix (g)** | 200 | 200 |
| **Amount of cysteamine- containing composition (formula I) in specific premix (wt%)** | 10 | 10 |
| **Total diet per day (g)** | 46800 | 44000 |

**Table 5:** Diets of the Group II dairy cows

| Ingredients | Before April 30 | On or After April 30 |
|---|---|---|
| **Basal feed (g)** | 46600 | 43800 |
| **Specific premix (g)** | 200 | 200 |
| **Amount of cysteamine- containing composition (formula II) in specific premix (wt%)** | 20 | 20 |
| **Total diet per day (g)** | 46800 | 44000 |

**Table 6:** Diets of the Group III (control) dairy cows

| Ingredients | Before April 30 | On or After April 30 |
|---|---|---|
| **Basal feed (g)** | 46600 | 43800 |
| **Specific premix (g)** | 180 | 180 |
| **Amount of cysteamine- containing composition (formula III) in specific premix (wt%)** | 0 | 0 |

(continued)

| Ingredients | Before April 30 | On or After April 30 |
|---|---|---|
| **Total diet per day (g)** | 46780 | 43980 |

The cysteamine-containing composition being in mini-pill form comprised about 30wt% cysteamine together with other ingredients including cyclodextrin which served as a stabilizer. The content of cyclodextrin in the composition was 10wt%. The composition was prepared by Walcom BioChemicals Industry Limited.

To form the final feeds, the appropriate formula of the specific premix was mixed with the suitable basal feed.

Studies have shown that in practice, the cysteamine-containing composition may contain 1 to 95wt% cysteamine. In any event, it is preferred that the final feed is adjusted to contain approximately 400 to 1000ppm of the composition. Alternatively, the final feed may be adjusted to contain approximately 120 to 300ppm of cysteamine.

In its dry state, the feed may comprise 1000 to 2147ppm of the composition and 200 to 650ppm of cysteamine. The composition used in the experiment comprised 10wt% cyclodextrin which is mentioned above. However, depending on the actual amount of cysteamine used in preparing the composition, the composition may contain 1 to 80wt% cyclodextrin, as well as other ingredients which may include a bulking agent, a disintegration agent and a solid coated carrier. The composition is in the form of mini-pill having a multi-layer structure. The composition thus remains relatively stable at room temperature conditions and un-dissolved at a pH as low as 1.5 to 3.5 (such as in a stomach environment) after it has been ingested by the animal. However, the carrier is made of a coating material which is soluble in a higher pH environment such as in the intestines.

Procedure

All three groups of cows were kept together under the same conditions which included tether feeding and automatic drinking. The cows were allowed four hours of exercising and feeding every day. The cows were fed three times a day. During each feeding time, the cows were firstly fed with roughage and then with the respective diets (see Tables 4 to 6). The consumption of roughage was not restricted but was monitored. The cows were milked three times a day at 0600, 1400 and 2100.

During the experiment, data of milk yield expressed in its original amount and in 3.5wt% FCM, fat content and protein content of the milk as well as the number of somatic cells in the milk were recorded and determined.

Results and discussion

Fig. 1 shows three curves representing 3.5wt% FCM yield. As clearly shown in the figure, the Group II cows generally had the highest 3.5wt% FCM yield among the three groups of cows throughout the experiment. The Group I cows also had a generally higher 3.5wt% FCM yield as compared to the Group III cows.

Table 7 below shows the data of average original milk yield of the three groups of cows. It is calculated that after feeding on the respective diets including the cysteamine-containing composition, the Groups I and II cows on average increased lactation by 4.86% and 6.88% ($p<0.05$) when compared to the Group III cows. Statistically, these are significant increases.

**Table 7**: Comparison of average milk yield (kg/day)

| Period | Group I | Group II | Group III |
|---|---|---|---|
| **Before trial** | 27.5 ± 4.27a | 27.4 ± 4.02a | 27.5 ± 4.1 a |
| **1$^{st}$ to 10$^{th}$ day** | 28.99 ± 0.43a | 29.5 ± 0.60a | 27.29 ± 0.72b |
| **11$^{th}$ to 20$^{th}$ day** | 27.98 ± 0.37a | 28.9 ± 0.24a | 27.17 ± 0.39b |
| **21$^{st}$ to 30$^{th}$ day** | 28.05 ± 0.59a | 28.51 ± 0.75a | 26.71 ± 0.61 b |
| **31$^{st}$ to 40$^{th}$ day** | 27.23 ± 0.77a | 27.48 ± 0.52a | 25.85 ± 0.53b |
| **1$^{st}$ to 40$^{th}$ day** | 28.06 ± 0.84a | 28.60 ± 0.91 a | 26.76 ± 0.79b |
| **41$^{st}$ to 49$^{th}$ day (stop feeding cysteamine-containing diet)** | 26.09 ± 0.67a | 26.12 ± 0.76a | 25.42 ± 0.40b |
| (Note: In each row, the figures labeled with same letters means that there is no significant difference statistically, i.e. $p>0.05$; the figures labeled with different letters means that there is significant difference statistically, i.e. $p<0.05$.) | | | |

For the sake of comparison, the original milk yield of the three groups cows shown in Table 7 has been converted to 3.5wt% FCM yield which is shown in Table 8 below. The following formula is used to convert original milk yield to its corresponding FCM (3.5wt%) yield.

$$\text{Equivalent FCM} = (0.44 \times \text{daily milk yield}) + (16 \times \text{daily milk yield} \times \text{fat content rate})$$

**Table 8**: Comparison of average FCM (3.5%wt) yield (kg/day)

| Period | Group I | Group II | Group III (control) |
|---|---|---|---|
| **Before trial** | 28.64 ± 5.25a | 28.73 ± 5.78a | 28.98 ± 4.97a |
| **1st to 10th day** | 29.51 ± 0.49a | 31.07 ± 0.69b | 28.73 ± 0.78c |
| **11th to 20th day** | 30.56 ± 0.41 a | 31.26 ± 0.26a | 29.22 ± 0.42b |
| **21st to 30th day** | 30.46 ± 0.67a | 30.37 ± 0.81a | 28.60 ± 0.65b |
| **31st to 40th day** | 29.23 ± 0.34a | 28.84 ± 0.52a | 27.52 ± 0.59b |
| **1st to 40th day** | 29.94 ± 0.63a | 30.39 ± 0.98a | 28.52 ± 0.68b |
| **41st to 49th day(stop feeding cysteamine-containing diet)** | 27.10 ± 0.85a | 25.91 ± 0.78b | 25.82 ± 0.61b |
| (Note: In each row, the figures labeled with same letters means that there is no significant difference statistically, i.e. p>0.05; the figures labeled with different letters means that there is significant difference statistically, i.e. p<0.05.) | | | |

It is calculated that after feeding on the respective diets including the cysteamine-containing composition, the Groups I and II cows had a 4.98% (p<0.01) and 6.56% (p<0.01) higher FCM (3.5wt%) yield when compared to the Group III cows. Statistically, these are significant increases.

As illustrated above, the Groups I and II cows significantly improved their lactation in terms of its original milk yield and 3.5wt% FCM yield after feeding on cysteamine-containing diets.

It is to be noted that after each calving, the lactation of a cow normally lasts for approximately 300 days. The above experimental data indicates that administering cysteamine/cysteamine-containing composition to dairy cows at least heightens lactation. It appears that cysteamine can also prolong lactation since when the lactation is maintained at a higher level for a longer time, the lactation should be prolonged to a certain extent, as shown by the curve representing the milk yield of the Group I cows in Fig. 1.

Table 9 below shows the data of average fat content in the milk produced by the three groups of cows.

**Table 9**: Comparison of average fat content (wt%) in milk

| Period | Group I | Group II | Group III (control) |
|---|---|---|---|
| **Before trial** | 3.76 ± 0.41a | 3.78 ± 0.37a | 3.84 ± 0.40b |
| **1st to 10th day** | 3.62 ± 0.39a | 3.86 ± 0.26b | 3.84 ± 0.26b |
| **11th to 20th day** | 4.10 ± 0.42a | 4.00 ± 0.24a | 3.99 ± 0.23a |
| **21st to 30th day** | 4.06 ± 0.42a | 3.90 ± 0.21b | 3.95 ± 0.16b |
| **31st to 40th day** | 4.02 ± 0.45a | 3.80 ± 0.22b | 3.91 ± 0.20c |
| **1st to 40th day** | 3.95 ± 0.45a | 3.89 ± 0.23a | 3.92 ± 0.21a |
| **41st to 49th day (stop feeding cysteamine-containing diet)** | 3.69 ± 0.53a | 3.53 ± 0.20a | 3.68 ± 0.24a |
| (Note: In each row, the figures labeled with same letters means that there is no significant difference statistically, i.e. p>0.05; the figures labeled with different letters means that there is significant difference statistically, i.e. p<0.05.) | | | |

Before the experiment, there is no significant difference of milk fat content among the three groups of cows ($p>0.05$, see Table 1). It is calculated that after feeding on the respective diets including the cysteamine-containing composition, the milk produced by the Group I cows had an average of 0.77% ($p<0.05$) higher fat content than that of the Group III cows. It is also calculated that after feeding on the respective diets including the cysteamine-containing composition, the milk produced by the Group II cows had an average of 0.77% ($p<0.5$) lower fat content than that of the Group III cows during the experiment, although the difference is not significant statistically. As illustrated above, there is no significant difference in fat content in milk produced by the Groups I and II cows after their diets were added with cysteamine/cysteamine-containing composition. This indicates that the quality of milk produced by cows feeding on a cysteamine-containing diet can at least be maintained if not increased.

It is however to be noted that during the first ten-day period of the experiment, the Group I cows appeared to have reacted more slowly to its cysteamine-containing diet in terms of the fat content in its milk in comparison to the Group II cows. However, the fat content in the milk produced by the Group I cows had risen rapidly and peaked at 4.10wt% during the period of 11$^{st}$ to 20$^{th}$ day of the experiment. To the contrary, the Group II cows appeared to have reacted relatively more rapidly to its cysteamine-containing diet in the first ten-day period. In particular, the fat content was 3.78wt% before the experiment but rose to 3.86wt% in the first ten-day period of the experiment, and the fat content peaked at 4.00wt%. It is to be noted that the milk produced by the Group II cows had the lowest fat content among the three groups when all the cows were stopped feeding on the respective cysteamine-containing diets. It is thus indicated in the experiment that cysteamine or a cysteamine-containing composition when administered at an appropriate dose and/or a particular lactation stage can increase the content of milk fat in the milk.

Table 10 below shows the data of average milk protein content in milk produced by the three groups of cows.

**Table 10:** Comparison of average milk protein content (g/cow/day)

| **Period** | **Group I** | **Group II** | **Group III (control)** |
|---|---|---|---|
| **Before trial** | 856.6 ± 109a | 864.7 ± 159.6a | 869.8 ± 112.7a |
| **1$^{st}$ to 10$^{th}$ day** | 926.9 ± 89.4a | 941.8 ± 109.2a | 904.7 ± 116.3b |
| **11$^{th}$ to 20$^{th}$ day** | 911.9 ± 80.1a | 917.6 ± 126.8a | 875.8 ± 82.7b |
| **21$^{st}$ to 30$^{th}$ day** | 928.3 ± 75.8a | 903.4 ± 120.7b | 878.2 ± 106.9c |
| **31$^{st}$ to 40$^{th}$ day** | 912.8 ± 69.5a | 868.7 ± 121.3b | 875.2 ± 103.2b |
| **1$^{st}$ to 40$^{th}$ day** | 920.1 ± 28.8a | 907.9 ± 30.6a | 883.5 ± 24.2b |
| **41$^{st}$ to 49$^{th}$ day (stop feeding cysteamine-containing diet)** | 877.1 ± 79.4a | 830.9 ± 116.5b | 850.7 ± 89.0c |
| (Note: In each row, the figures labeled with same letters means that there is no significant difference statistically, i.e. $p>0.05$; the figures labeled with different letters means that there is significant difference statistically, i.e. $p<0.05$.) | | | |

Before the experiment, the protein content in milk (g/day/cow) produced by the Group III cows was about 869.8g which was slightly higher than that of the Groups I and II cows, although the difference was not significant statistically. After the Groups I and II cows were fed with the respective cysteamine-containing diets, the protein content in their milk increased significantly by 4.14% and 2.76% respectively ($p<0.05$) as compared to the Group III cows. After the Groups I and II cows stopped feeding on the respective cysteamine-containing diets, the protein content reduced close to the pre-experiment levels. It is therefore illustrated that feeding on cysteamine-containing diet not only increases original milk yield, FCM yield and milk fat content but also milk protein content.

Table 11 below shows the data of the number of somatic cells in the milk produced by the three groups of cows.

**Table 11:** Comparison of somatic cells ($x10^3$/ml) in milk

| **Period** | **Group I** | **Group II** | **Group III (control)** |
|---|---|---|---|
| **Before trial** | 91.7 ± 64.9a | 86.3 ± 55.3a | 84.9 ± 64.7a |
| **1$^{st}$ to 10$^{th}$ day** | 96.6 ± 92.8a | 102.0 ± 78.0a | 116.9 ± 129.0a |
| **11$^{th}$ to 20$^{th}$ day** | 90.0 ± 82.9a | 87.2 ± 64.9a | 59.9 ± 33.0b |
| **21$^{st}$ to 30$^{th}$ day** | 122.8 ± 113.3a | 89.8 ± 60.9b | 69.5 ± 42.7b |
| **31$^{st}$ to 40$^{th}$ day** | 155.2 ± 155.1a | 92.1 ± 58.4b | 79.0 ± 59.7b |

(continued)

| Period | Group I | Group II | Group III (control) |
|---|---|---|---|
| **$1^{st}$ to $40^{th}$ day** | 116.1 ± 113.0a | 92.8 ± 63.6b | 81.3 ± 76.3b |
| **$41^{st}$ to $49^{th}$ day** | 115.2 ± 115.9a | 96.2 ± 54,5b | 93.9 ± 45.5b |
| (Note: In each row, the figures labeled with same letters means that there is no significant difference statistically, i.e. p>0.05; the figures labeled with different letters means that there is significant difference statistically, i.e. p<0.05.) | | | |

After feeding on the respective cysteamine-containing diets, the milk produced by the Groups I and II cows had a higher number of somatic cells. However, the difference remained at an acceptable safety level.

In the experiment, Formula II of the specific premix comprised twice as much the cysteamine-containing composition. In other words, the Group II cows were fed with twice as much cysteamine/cysteamine-containing compound as compared to the Group I cows. While the milk yield did increase as compared to that of the Group III cows, the increase was not twice as much as compared to the Group I cows. It is believed there is a certain physiological limit a cow can react to cysteamine. In the event that a relatively high amount of cysteamine were taken by a cow, the physiology would still not react exceeding its limit. This is desirable as the dosage of the cysteamine-containing composition in the diet is not critical in that an overdose thereof is not dangerous to the animal. Thus, administering the cysteamine-containing composition in dairy cows is safe and easy to carry out.

It is to be noted that a final feed has a relatively high water content (e.g. 10 to 60wt% water content) and the precise concentrations of cysteamine and cysteamine-containing composition may thus vary depending on the water content thereof and the humidity of the surroundings. In this regard, it is worthwhile to express a workable range of the concentration of cysteamine and cysteamine-containing composition in the final feed based on its dry weight (state). For example, studies have determined that the concentration of 1000 to 2174ppm of the cysteamine-containing composition used in the above examples in the final feed based on its dry weight will produce the similar results in increasing lactation as shown in the examples. Similarly, it is calculated that the concentration of 200 to 650ppm of cysteamine in the final feed based on its dry weight will likewise produce similar results.

It is also to be noted that while the administration of cysteamine and the cysteamine-containing composition to the diary cows are performed via its diet, it is envisaged that cysteamine and the cysteamine-containing composition may likewise be fed to the dairy cows in the form of pills, tablets or other suitable state separately or together with a food material and this would produce the same effect. Studies have shown that the effective amount of cysteamine administered to raise the dairy cows may be 5.64 to 12.71g per cow per day. The effective amount of cysteamine-containing composition may be 18.79 to 42.36g per cow per day.

Experiment 2

Background information

100 black & white dairy cows were used in the experiment. Before the experiment, the average body weight of the dairy cows was about 600kg; the average lactation period of the cows was about $135^{th}$ day and the average milk production was about 35kg. The quality of the milk produced by the cows prior to the experiment was similar. The cows were not administered with any cysteamine products beforehand.

The cows were equally divided into one test group and one respective control group. Each of the test and control groups are further divided into four sub-groups during their $19^{th}$ week of lactation, namely Groups I to IV, according to their actual milk production, calving history and stage of lactation. The prior milk production (MP) of the Groups I to IV cows were MP ≤ 30kg, 30kg < MP ≤ 35kg, 35kg < MP ≤ 40kg, 40kg < MP respectively.

Procedure and materials

The experiment was carried out during the $20^{th}$ to $32^{nd}$ week of lactation of the cows. The actual experiment was preceded by a two-day adaptation period. At different periods during the experiment, the test sub-groups of cows were administered with different amounts of a cysteamine-containing composition via a basal feed. The composition is identical to that used in Experiment 1. In particular, the composition comprises substantially 30%wt cysteamine.

The control group of cows was fed with the same basal feed as the test group of cows. The amount of basal feed used was adjusted according to the actual milk production at the time.

All the cows were fed three times daily. During each feeding time, the cows were fed firstly with roughage and then the basal feed mixed with or without the composition. The cows were milked three times daily at 0730, 1430 and 2130.

The milk collected was measured for its quantity and analyzed for its milk fat content and milk protein content.

Results and discussion

Tables 12 to 16 below summarize the experimental data of Experiment 2.

**Table 12**: Content of milk fat (wt%) produced by the test and control groups of cows

| CCC | 20 | | 30 | | 40 | | 60 | | mean | |
|---|---|---|---|---|---|---|---|---|---|---|
| Sub- group\ Group | T | C | T | C | T | C | T | C | T | C |
| I | 3.67± 0.70 | 3.77± 0.65 | 3.66± 0.79 | 3.91± 0.68 | 3.63± 0.72 | 3.82± 0.57 | 3.52± 0.43 | 3.45± 0.77 | 3.69± 0.09* | 3.73± 0.20 |
| II | 3.95± 0.41 | 3.81± 0.49 | 3.87± 0.45 | 3.74± 0.60 | 3.98± 0.43* | 3.78± 0.47 | 3.62± 0.45 | 3.45± 0.77 | 3.89± 0.15* | 3.73± 0.16 |
| III | 3.58± 0.38 | 3.62± 0.58 | 3.51± 0.56 | 3.54± 0.66 | 3.73± 0.46 | 3.86± 0.53 | 3.65± 0.47 | 3.64± 0.33 | 3.64± 0.15 | 3.70± 0.17 |
| IV | 3.95± 0.38# | 3.62± 0.37 | 3.82± 0.38* | 3.54± 0.46 | 3.92± 0.48** | 3.48± 0.55 | 3.89± 0.43* | 3.37± 0.41 | 3.89± 0,12** | 3.50± 0.12 |
| | mean | | mean | | mean | | mean | | mean | |
| | 3.80 ± 0.49 | 3.72 ± 0.51 | 3.73 ± 0.57 | 3.67 ± 0.61 | 3.84 ± 0.53 | 3.75 ± 0.53 | 3.65 ± 0.45# | 3.50 ± 0.47 | 3.77 ± 0.11 | 3.66 ± 0.12 |
| (Note: CCC = Cysteamine-containing composition, g per cow per day (g/c/d); T = Test group; C = Control group; *p<0.05; ** P<0.01; #0.05<P<0.15) | | | | | | | | | | |

Referring to Table 12, it is shown that after administered with a feed containing the cysteamine-containing composition during the experiment, there was an average increase of milk fat content in the milk produced by the test cows by about 3.01% (=[3.77wt% - 3.66wt%]/3.66wt% x 100%). In the test Group II of cows, the average increase of milk fat content was about 4.29% (=[3.89wt% - 3.73wt%]/3.73wt% x 100%). In the test Group IV of cows, the average increase of milk fat content was more significant by about 11.14% (=[3.89wt%-3.50wt%]/3.50wt% x 100%). Since the Group IV of cows were the cows with the highest milk yield before the experiment, it is shown that the effect of cysteamine on milk fat content is more prominent on the dairy animals with already higher milk production.

Referring to the four test sub-groups of cows administered with 20g/c/d, 30g/c/d, 40g/c/d and 60g/c/d of the composition respectively, it is calculated that the increase in milk fat content in the milk produced are 2.15% 1.63%, 2.40% and 4.29% respectively.

**Table 13:** Content of milk protein (wt%) produced by the test and control groups of cows

| CCC | 20 | | 30 | | 40 | | 60 | | mean | |
|---|---|---|---|---|---|---|---|---|---|---|
| Sub- group\ Group | T | C | T | C | T | C | T | C | T | C |
| I | 3.10 ± 2.33# | 3.41 ± 0.33 | 3.06 ± 0.26* | 3.32 ± 0.36 | 2.96 ± 0.17* | 3.22 ± 0.35 | 2.99 ± 0.15 | 2.73 ± 0.81 | 3.01 ± 0.06* | 3.21 ± 0.05 |
| II | 3.12 ± 0.28* | 2.91 ± 0.22 | 3.14 ± 0.21** | 2.94 ± 0.24 | 3.00 ± 0.25# | 2.93 ± 0.21 | 3.00 ± 0.21 | 3.01 ± 0.22 | 3.06 ± 0.08** | 2.94 ± 0.04 |
| III | 2.96± 0.28 | 2.94± 0.25 | 3.01± 0.34 | 2.98± 0.34 | 2.91± 0.25 | 2.88± 0.27 | 2.92± 0.21 | 2.89± 0.22 | 2.95± 0.06 | 2.92± 0.05 |
| IV | 2.84± | 2.63± | 2.92± | 2.67± | 2.86± | 2.66± | 2.88± | 2.76± | 2.88± | 2.67± |

(continued)

| CCC | 20 | | 30 | | 40 | | 60 | | mean | |
|---|---|---|---|---|---|---|---|---|---|---|
| Sub- group\ Group | T | C | T | C | T | C | T | C | T | C |
| | 0.27* | 0.13 | 0.26** | 0.16 | 0.26** | 0.19 | 0.27 | 0.21 | 0.05** | 0.06 |
| | mean | | mean | | mean | | mean | | mean | |
| | 3.03± 2.08* | 2.92± 0.03 | 3.00± 0.29# | 2.92± 0.31 | 2.92± 0.24 | 2.90± 0.28 | 2.96± 0.21# | 2.90± 0.33 | 3.06± 0.17* | 2.92± 0.04 |
| (Note: CCC = Cysteamine-containing composition, g per cow per day (g/c/d); T = Test group; C = Control group; *p<0.05; **P<0.01; #0.05<p<0.15) | | | | | | | | | | |

Table 13 shows that the average increase of the milk protein content in milk produced by the test cows was about 4.79% (=[3.06wt% - 2.92wt%]/2.92wt% x 100%). In particular, the increase of the milk protein content in milk produced by the test Group II of cows was about 4.08% (=[3.06wt%-2.94wt%] /2.94wt% x 100%). The increase of the milk protein content in milk produced by the test Group IV of cows was more significant by about 7.87% (=[2.88wt% - 2.67wt%]/2.67wt% x 100%). Since the Group IV cows were the cows with the highest yield of milk production before the experiment, it is shown that the effect of cysteamine on milk protein content is more prominent on the dairy animals with already higher milk production.

Referring to the dosage effects of the composition on milk protein content, the dose of 20g/c/d increased the milk protein content significantly in the test sub-groups of cows by about 3.77% (=[3.03wt% - 2.92wt%]/2.92wt% x 100%).

**Table 14:** Production of 3.5wt% FCM milk by the test and control groups of cows

| CCC | 20 | | 30 | | 40 | | 60 | | mean | |
|---|---|---|---|---|---|---|---|---|---|---|
| Sub-group\ Group | T | C | T | C | T | C | T | C | T | C |
| I | 29.4 ±3.4 | 28.3 ±3.7 | 28.9 ±4.4 | 25.4 ±5.0 | 25.9 ±4.5 | 22.4 ±6.2 | 22.6 ±3.3 | 19.9 ±7.0 | 26.7 ±3.2 | 24.0 ±5.2 |
| II | 34.9 ±3.4 | 35.2 ±3.1 | 33.4 ±4.7 | 33.0 ±4.0 | 30.9 ±5.0 | 29.9 ±4.6 | 26.6 ±5.8 | 26.0 ±5.2 | 31.0 ±3.2 | 31.4 ±3.4 |
| III | 37.8 ±3.1 | 37.8 ±4.6 | 35.5 ±4.3 | 35.5 ±5.9 | 33.7 ±5.6 | 34.1 ±5.9 | 29.7 ±5.5 | 29.4 ±6.1 | 34.2 ±4.0 | 34.2 ±4.7 |
| IV | 45.0 ±4.8 | 43.6 ±4.6 | 42.1 ±2.0 | 40.5 ±6.5 | 38.7 ±3.4# | 34.2 ±7.3 | 35.0 ±2.0# | 30.9 ±5.9 | 40.2 ±12.0 | 37.3 ±5.8 |
| | mean | | mean | | Mean | | mean | | mean | |
| | 36.1 ±25.8 | 36.7 ±5.6 | 34.4 ±5.8 | 34.2 ±6.4 | 31.8 ±6.2 | 31.0 ±6.6 | 27.9 ±6.2 | 27.1 ±6.4 | 32.4 ±5.6 | 32.3 ±5.7 |
| (Note: CCC = Cysteamine-containing composition, g per cow per day (g/c/d); T = Test group; C = Control group; #0.05<p<0.15) | | | | | | | | | | |

Table 14 shows that the test Groups I and IV of cows increased their 3.5wt% FCM milk yield very significantly by 11.25% (=[26.7kg/day - 24.0kg/day]/24.0kg/day x 100%) and 7.7% (=[40.2kg/day - 37.3kg/day]/37.3kg/day x 100%) respectively, while in the test Groups II and III of cows, the production of 3.5wt% FCM was not altered significantly as compared to that of the respective control groups. Since the test Group I of cows were the cows with the lowest milk yield before the experiment, it is shown that the positive effect of cysteamine on 3.5% FCM yield is more prominent on dairy animals with lower FCM yield.

**Table 15:** Production of original milk (kg/day) by the test and control groups of cows

| CCC | 20 | | 30 | | 40 | | 60 | | Mean | |
|---|---|---|---|---|---|---|---|---|---|---|
| **Sub- group\ Group** | **T** | **C** | **T** | **C** | **T** | **C** | **T** | **C** | **T** | **C** |
| **I** | 28.4 ±0.5# | 25.8 ±1.3 | 28.1 ±0.6* | 23.7 ±1.3 | 25.2 ±0.8# | 21.1 ±1.9 | 22.0 ±0.8* | 19.7 ±2.1 | 25.9 ±0.7** | 23.1 ±0.9 |
| **II** | 32.2 ±0.6# | 33.6 ±0.5 | 31.2 ±0.7 | 31.7 ±0.5 | 28.6 ±0.8 | 28.6 ±0.8 | 25.9 ±1.0 | 26.0 ±0.9 | 29.6 ±0.5 | 29.9 ±0.6 |
| **III** | 37.2 ±0.6 | 36.8 ±1.0 | 35.4 ±0.7 | 35.2 ±1.2 | 32.4 ±1.1 | 32.2 ±1.4 | 28.9 ±1.2 | 28.6 ±1.4 | 33.5 ±0.7 | 33.3 ±0.7 |
| **IV** | 42.0 ±0.7 | 43.0 ±0.7 | 40.1 ±1.0 | 40.2 ±1.9 | 36.3 ±1.3 | 34.0 ±1.9 | 33.1 ±1.2 | 31.3 ±1.6 | 37.9 ±1.0# | 36.8 ±1.4 |
| | mean | | Mean | | Mean | | Mean | | Mean | |
| | 34.2 ±0.7 | 35.2 ±0.8 | 33.0 ±0.7 | 33.7 ±0.8 | 30.1 ±0.7 | 29.6 ±0.8 | 27.1 ±0.7 | 26.9 ±0.8 | 31.1 ±0.3 | 31.0 ±0.4 |
| (Note: CCC = Cysteamine-containing composition, g per cow per day (g/c/d); T = Test group; C = Control group; *p<0.05; **p<0.01; #0.05<p<0.15) | | | | | | | | | | |

**Table 16:** Production of milk protein (kg/day) by the test and control groups of cows

| CCC | 20 | | 30 | | 40 | | 60 | | mean | |
|---|---|---|---|---|---|---|---|---|---|---|
| **Sub- group\ Group** | **T** | **C** | **T** | **C** | **T** | **C** | **T** | **C** | **T** | **C** |
| **I** | 0.88 ±0.08 | 0.88 ±0.17 | 0.86 ±0.09 | 0.79 ±0.16 | 0.74 ±0.06 | 0.69 ±0.19 | 0.67 ±0.06 | 0.56 ±0.27 | 0.79 ±0.12# | 0.71 ±0.18 |
| **II** | 1.00 ±0.11 | 0.97 ±0.10 | 0.93 ±0.25 | 0.93 ±0.10 | 0.86 ±0.13 | 0.83 ±0.10 | 0.77 ±0.13 | 0.78 ±0.12 | 0.89 ±0.12 | 0.88 ±0.08 |
| **III** | 1.10 ±0.12 | 1.08 ±0.14 | 1.06 ±0.14 | 1.05 ±0.16 | 0.94 ±0.14 | 0.93 ±0.17 | 0.84 ±0.13 | 0.83 ±0.17 | 0.99 ±0.11 | 0.97 ±0.14 |
| **IV** | 1.19 ±0.09# | 1.13 ±0.07 | 1.17 ±0.08# | 1.07 ±0.12 | 1.04 ±0.09* | 0.90 ±0.10 | 0.95 ±0.07# | 0.86 ±0.10 | 1.09 ±0.11 | 0.99 ±0.14 |
| | mean | | Mean | | Mean | | Mean | | Mean | |
| | 1.03 ±0.15 | 1.02 ±0.14 | 1.01 ±0.15 | 0.98 ±0.15 | 0.89 ±0.15 | 0.85 ±0.15 | 0.78 ±0.17 | 0.80 ±0.14 | 0.93 ±0.15 | 0.91 ±0.14 |
| (Note: CCC = Cysteamine-containing composition, g per cow per day (g/c/d); T = Test group; C = Control group; #0.05<p<0.15) | | | | | | | | | | |

Table 15 shows that the original milk yields of the test Group I of cows increased very significantly by 12.12% (=[25.9kg/day - 23.1kg/day]/23.1kg/day x 100%).

Table 16 shows that the actual quantity of milk protein produced by the test Group I of cows increased by 11.27% (=[0.79kg/day - 0.71kg/day]/0.71kg/day x 100%). However, the data in Tables 12 to 14 is more representative in illustrating the positive effects of cysteamine on improving lactation of dairy animals.

**Table 17:** Effect of the composition on feed to milk ratio

| CCC | 20 | | 30 | | 40 | | 60 | | mean | |
|---|---|---|---|---|---|---|---|---|---|---|
| Sub- group\ Group | T | C | T | C | T | C | T | C | T | C |
| I | 0.52 ±0.01 | 0.56 ±0.03 | 0.54 ±0.09 | 0.60 ±0.08 | 0.54 ±0.06* | 0.68 ±0.10 | 0.58 ±0.08 | 0.68 ±0.14 | 0.54 ±0.06** | 0.63 ±0.11 |
| II | 0.46 ±0.04* | 0.44 ±0.03 | 0.47 ±0.04 | 0.46 ±0.04 | 0.50 ±0.05 | 0.50 ±0.06 | 0.51 ±0.08 | 0.50 ±0.09 | 0.48 ±0.06 | 0.48 ±0.06 |
| III | 0.39 ±0.02 | 0.40 ±0.03 | 0.41 ±0.03 | 0.42 ±0.05 | 0.42 ±0.06 | 0.43 ±0.06 | 0.46 ±0.08 | 0.47 ±0.10 | 0.41 ±0.04 | 0.43 ±0.05 |
| IV | 0.35 ±0.02 | 0.34 ±0.02 | 0.36 ±0.03 | 0.37 ±0.05 | 0.37 ±0.03 | 0.40 ±0.06 | 0.40 ±0.04 | 0.42 ±0.05 | 0.38 ±0.02 | 0.39 ±0.04 |
| | mean | | mean | | mean | | mean | | mean | |
| | 0.45 ±0.07 | 10.43 ±0.06 | 0.45 ±0.08 | 10.45 ±0.08 | 0.47 ±0.07 | 0.49 ±0.10 | 0.49 ±0.09 | 10:50 ±0.12 | 0.46 ±0.07 | 0.47 ±0.09 |
| (Note: CCC = Cysteamine-containing composition, g per cow per day (g/c/d); T = Test group; C = Control group) | | | | | | | | | | |

Table 17 above shows that the feed to milk ratio is generally not affected whether or not the cows are administered with the composition. This is important from a cost point of view.

Experiment 3

Background information

Thirty-two black & white dairy cows were used in the experiment and were randomly divided into four groups, namely test Groups I to III, and a control group. Each group had eight cows. The characteristics of milk produced by the cows before the experiment is summarized in Table 17 below.

**Table 18:** Dairy performance of the four groups of cows before the experiment

| Characteristics\ Group | I | II | III | Control |
|---|---|---|---|---|
| **Number of cows** | 8 | 8 | 8 | 8 |
| **Calving number** | 1 | 1 | 1 | 1 |
| **Day of lactation** | 20 | 20 | 20 | 20 |
| **Daily milk production, kg/cow/day** | 26.90 ± 1.91 | 26.84 ± 4.51 | 26.76 ± 3.77 | 26.74 ± 4.47 |
| **3.5wt% FCM, kg/cow/day** | 28.22 ± 2.37 | 29.81 ± 2.48 | 28.35 ± 3.99 | 29.73 ± 3.35 |
| **Milk fat content, wt%** | 3.90 ± 0.56 | 4.19 ± 0.58 | 3.87 ± 0.93 | 4.20 ± 0.78 |
| **Daily milk protein production, g/cow/day** | 698.88 ± 63.64 | 754.59 ± 49.45 | 744.93 ± 52.86 | 713.29 ± 76.97 |
| **Number of somatic cells in milk, $10^3$/ml** | 1097.4 ± 1070.2 | 803.00 ± 1113.4 | 126.90 ± 87.10 | 99.90 ± 136.50 |

Procedure and materials

The cysteamine-containing composition used was the same composition in Experiments 1 and 2 comprising substantially 30wt% cysteamine.

There were three stages in the experiment. In the first stage of the experiment, the feed for the test Groups I to III of cows was mixed with the composition such that each cow in the three test groups was fed with 20g, 30g and 40g of the composition respectively daily. No cysteamine-containing composition was mixed in the feed for the control

group of cows. The first stage of the experiment lasted for thirty-seven days and was preceded by a three-day adaptation period.

During the second stage of the experiment, the composition was not added to the feed for the three test groups of cows. However, each cow in the control group was fed with 20g of the composition daily via its feed. The second stage of the experiment lasted for seven days.

During the third stage of the experiment, the three test groups of cows were fed with the feed mixed with the composition such that 20g of the composition was taken by each cow daily. Each cow in the control group was fed with 40g of the composition every other day via the feed. The third stage of the experiment lasted for seven days.

The daily milk yield, milk fat content, milk protein content and somatic cells in milk were measured and recorded. The cows were fed three times a day and milked also three times a day.

Results and discussion

Tables 19 to 24 summarize the data from the experiment.

**Table 19:** Effect of the composition on original milk yield (kg/cow/day)

| Time\Group | Test Group I | Test Group II | Test Group III | Control Group |
|---|---|---|---|---|
| **Before experiment** | 26.90 ± 1.91$^{a}$ | 26.84 ± 4.51$^{ab}$ | 26.76 ± 3.77$^{abc}$ | 26.74 ± 4.47$^{abcd}$ |
| **Stage one** | | | | |
| **$1^{st}$ to $10^{th}$ day** | 27.52 ± 0.52$^{a}$ | 28.05 ± 0.20$^{b}$ | 27.40 ± 0.56$^{ac}$ | 27.89 ± 0.44 $^{abcd}$ |
| **$11^{th}$ to $20^{th}$ day** | 28.87 ± 0.34$^{a}$ | 26.39 ± 0.91 $^{b}$ | 26.59 ± 0.26$^{bC}$ | 27.61 ± 0.59$^{bd}$ |
| **21 $^{th}$ to $30^{th}$ day** | 28.45 ± 0.53$^{a}$ | 26.77 ± 0.92$^{B}$ | 26.57 ± 0.23$^{bc}$ | 26.49 ± 0.20$^{bcD}$ |
| **$31^{st}$ to $37^{th}$ day** | 27.64 ± 0.84$^{a}$ | 27.24 ± 0.42$^{ab}$ | 26.09 ± 0.20$^{ac}$ | 27.08 ± 1.12$^{abcd}$ |
| **Average: $1^{st}$ to $37^{th}$ day** | 28.14 ± 0.74 $^{A}$ | 27.15 ± 0.91$^{B}$ | 26.76 ± 0.58$^{BC}$ | 27.27 ± 0.76$^{BCD}$ |
| **Stage two** | 26.95 ± 0.52$^{a}$ | 25.94 ± 1.07$^{ab}$ | 25.44 ± 0.13$^{bC}$ | 25.09 ± 0.64$^{bcd}$ |
| **Stage three** | 25.51 ± 0.32$^{a}$ | | | 27.00 ± 0.42$^{B}$ |

(Note: In the table, same letters in a row means that the difference of their values is insignificant, i.e. p>0.05, different letters in a same row means that the difference of their values is significant p<0.05; different letter size in a column means their values are significant, i.e. p<0.01)

Table 19 above shows that the test Group I of cows increased their original milk yield by 7.41% (= [26.95 - 25.09]/25.09 x 100%) when compared to that of the cows in the control group. In particular, the increase in milk yield was more significant after the first ten-day period. For instance, the increase in milk yield of the test Group I of cows during the $11^{th}$ to $20^{th}$ day and $21^{st}$ to $30^{th}$ day was 4.56% (=[28.87 - 27.61]/27.61 x 100%) and 7.40% (=[28.45 - 26.49] /26.49 x 100%) respectively. It is thus illustrated that administering an appropriate dose of the composition to dairy animals is effective in increasing their original milk yield. In particular, the increase is more noticeable after an initial short period of the administration of the composition.

**Table 20**: Effect of the composition on quantity of milk fat (kg/cow/day) in milk

| Time\Group | Test Group I | Test Group II | Test Group III | Control Group |
|---|---|---|---|---|
| **Before the experiment** | 3.90±0.56$^{a}$ | 4.19 ± 0.58$^{ab}$ | 3.87 ± 0.93$^{abc}$ | 4.20 ± 0.78$^{abcd}$ |
| **Stage one** | | | | |
| **$1^{st}$ to $20^{th}$ day** | 3.63 ± 0.68$^{a}$ | 3.63 ± 0.61$^{ab}$ | 4.07 ± 0.54$^{abc}$ | 3.63 ± 0.44$^{abd}$ |
| **$21^{st}$ to $37^{th}$ day** | 4.26 ± 0.59$^{a}$ | 4.35 ± 0.78$^{ab}$ | 4.36 ± 0.76$^{abc}$ | 4.17± 0.54$^{abcd}$ |
| **Average: $1^{st}$ to $37^{th}$ day** | 3.95 ± 0.70$^{a}$ | 3.99 ± 0.77$^{ab}$ | 4.21 ± 0.65$^{abc}$ | 3.90 ± 0.55$^{abcd}$ |
| **Stage two** | 3.69 ± 0.53$^{a}$ | 3.69 ± 0.30$^{ab}$ | 3.73 ± 0.82$^{abc}$ | 3.58 ± 0.35$^{abcd}$ |

(continued)

| Time\Group | Test Group I | Test Group II | Test Group III | Control Group |
|---|---|---|---|---|
| Stage three | 3.94 ±0.31$^{a}$ | | | 3.98 ± 0.48$^{ab78}$ |
| (Note: In the table, same letters in a row means that the difference of their values is insignificant, i.e. p>0.05, different letters in a same row means that the difference of their values is significant p<0.05; different letter size in a column means their values are significant, i.e. p<0.01) | | | | |

Table 20 above shows that the milk fat in milk produced by the test Group III of cows increased by 7.95% (= [4.21-3.90]/3.90 x 100%) in stage one of the experiment. In particular, the increase in milk fat was particularly significant during the first twenty-day period of the experiment, i.e. 12.12% (= [4.07 - 3.63]/3.63 x 100%).

In stage two of the experiment, the test groups of cows were stopped feeding on the cysteamine-containing diet. However, the quantity of milk fat in the milk produced thereby was still higher when compared to that of the control group of cows. This illustrates that the effect of cysteamine lasted for some time after the administration of the composition was stopped.

**Table 21:** Effect of the composition on 3.5wt% FCM yield, kg/cow/day

| Time\Group | Test Group I | Test Group II | Test Group III | Control Group |
|---|---|---|---|---|
| Before experiment | 28.22 ± 2.37$^{a}$ | 29.81 ± 2.48$^{ab}$ | 28.35 ± 3.99$^{abc}$ | 29.73 ± 3.35$^{abc}$ |
| State one | | | | |
| 1$^{st}$ to 20$^{th}$ day | 29.76 ± 2.10$^{a}$ | 27.44 ± 2.63$^{B}$ | 28.94 ± 2.28$^{BC}$ | 28.74 ± 1.96$^{BCD}$ |
| 21$^{st}$ to 37$^{th}$ day | 30.37 ± 2.55$^{a}$ | 30.93 ± 3.40$^{ab}$ | 29.85 ± 3.17$^{abc}$ | 29.12 ± 2.28$^{abcd}$ |
| Average: 1$^{st}$ to 37$^{th}$ day | 30.07 ± 3.11$^{a}$ | 29.19 ± 3.30$^{ab}$ | 29.70 ± 2.17$^{abc}$ | 29.01 ± 2.17$^{abcd}$ |
| Stage two | 27.75 ± 2.30$^{a}$ | 26.72 ± 1.23$^{ab}$ | 26.37 ± 3.32$^{abc}$ | 25.41 ± 1.42$^{cd}$ |
| Stage three | 27.31 ± 1.25$^{a}$ | | | 29.08 ± 2.07$^{ab}$ |
| (Note: In the table, same letters in a row means that the difference of their values is insignificant, i.e. p>0.05, different letters in a same row means that the difference of their values is significant p<0.05; different letter size in a column means their values are significant, i.e. p<0.01) | | | | |

Table 21 above shows that the 3.5wt% FCM yield of the Group I of cows increased by 3.55% (=[29.76 - 28.74J/ 28.74 x 100%) during 1$^{st}$ to 20$^{th}$ day of the experiment and by 4.29% (=[30.37 - 29.12]/29.12 x 100%) during the 21$^{st}$ to 37$^{th}$ day of the experiment as compared to that of the control group of cows. This illustrates that the composition is also effective in increasing the 3.5% FCM yield.

In stage two of the experiment, although the three test groups of cows were stopped feeding on the cysteamine-containing diet, their 3.5wt% FCM yield was higher than the control group of cows that was feeding on the cysteamine-containing diet. This illustrates that the effect of cysteamine persists for a while after administration thereof was stopped and that it takes some time for cysteamine to take effect in the physiology of the animals.

**Table 22**: Effect of the composition on milk protein content (wt%)

| Time\Group | Test Group I | Test Group II | Test Group III | Control Group |
|---|---|---|---|---|
| Before experiment | 2.63 ± 0.24$^{a}$ | 2.81 ± 0.18$^{ab}$ | 2.78 ± 0.20$^{abc}$ | 2.67 ± 0.29$^{abcd}$ |
| State one | | | | |
| 1$^{st}$ to 20$^{th}$ day | 2.73 ± 0.21$^{a}$ | 2.85 ± 0.20$^{ab}$ | 2.85 ± 0.23$^{abc}$ | 2.81 ± 0.19$^{abcd}$ |
| 21$^{st}$ to 37$^{th}$ day | 2.70 ± 0.22$^{a}$ | 2.89 ± 0.14$^{ab}$ | 2.80 ± 0.22$^{abc}$ | 2.83 ± 0.10$^{abcd}$ |
| Average: 1$^{st}$ to 37$^{th}$ day | 2.72 ± 0.21$^{a}$ | 2.87 ± 0.17$^{b}$ | 2.83 ± 0.22$^{abc}$ | 2.82 ± 0.14$^{abcd}$ |
| Stage two | 2.65 ± 0.17$^{a}$ | 2.84 ± 0.09$^{b}$ | 2.72 ± 0.19$^{ab}$ | 2.76 ± 0.1$^{ac}$ |

(continued)

| Time\Group | Test Group I | Test Group II | Test Group III | Control Group |
|---|---|---|---|---|
| Stage three | 2.73 ± 0.18$^{a}$ | | 2.89 ± 0.11$^{b}$ | |
| (Note: In the table, same letters in a row means that the difference of their values is insignificant, i.e. p>0.05, different letters in a same row means that the difference of their values is significant p<0.05; different letter size in a column means their values are significant, i.e. p<0.01) | | | | |

**Table 23**: Effect of the composition on milk protein yield (g/cow/day)

| Time\Group | Test Group I | Test Group II | Test Group III | Control Group |
|---|---|---|---|---|
| Before experiment | 696.88 ± 63.64$^{a}$ | 754.59 ± 49.45$^{ab}$ | 744.93 ± 52.86$^{abc}$ | 713.29 ± 76.97$^{abcd}$ |
| State one | | | | |
| 1$^{st}$ to 20$^{th}$ day | 797.97 ± 61.72$^{a}$ | 766.37 ± 52.56$^{ab}$ | 757.05 ± 61.33$^{abc}$ | 789,33 ± 52.52$^{abcd}$ |
| 21$^{st}$ to 37$^{th}$ day | 731.09.± 58.2$^{a}$ | 786.03 ± 39.39$^{b}$ | 736.74 ± 56.89$^{ac}$ | 744.99 ± 25.83$^{acd}$ |
| Average: 1$^{st}$ to 37$^{th}$ day | 764.53 ± 58.24$^{a}$ | 776.15 ± 45.16$^{ab}$ | 746.90 ± 59.58$^{abc}$ | 767.16 ± 40.93$^{abcd}$ |
| Stage two | 714.85 ± 46.94$^{a}$ | 737.67 ± 23.49$^{ab}$ | 692.29 ± 47.25$^{ac}$ | 691.23 ± 23.95$^{acD}$ |
| Stage three | 696.00 ± 45.47$^{a}$ | | | 781.20 ± 30.82$^{b}$ |
| (Note: in the table, same letters in a row means that the difference of their values is insignificant, i.e. p>0.05, different letters in a same row means that the difference of their values is significant p<0.05; different letter size in a column means their values are significant, i.e. p<0.01) | | | | |

Table 23 above similarly shows the effect of the composition although the milk protein yield is expressed in gram per cow per day.

Table 23 shows that when the control group of cows was administered with 40g of cysteamine per cow every other day, the milk protein content increased by 5.86% (=[2.89wt% - 2.73wt%]/2.73wt% x 100%). This illustrates that administering cysteamine at a relatively high dose every other day can be more effective in increasing the milk protein yield than administering cysteamine at a relatively low dose daily.

**Table 24:** Number of somatic cells in milk produced by the cows (x10$^{3}$/ml)

| Time\Group | Test Group I | Test Group II | Test Group III | Control Group |
|---|---|---|---|---|
| Before experiment | 1097.4 ± 1070.2$^{a}$ | 803 ± 1113.4$^{ab}$ | 126.9 ± 87.1$^{abc}$ | 99.9 ± 136.5$^{abcd}$ |
| State one | | | | |
| 1$^{st}$ to 20$^{th}$ day | 296.25 ± 452.33$^{a}$ | 727.63 ± 917.02$^{ab}$ | 395.50 ± 643.44$^{abc}$ | 129.63 ± 152.56$^{acd}$ |
| 21$^{st}$ to 37$^{th}$ day | 360.25 ± 479.59$^{a}$ | 613.38 ± 556.70$^{ab}$ | 220.00 ± 348.65$^{abc}$ | 525.5 ± 917.59$^{abcd}$ |
| Average: 1$^{st}$ to 37$^{th}$ day | 328.25 ± 451.56$^{a}$ | 670.50 ± 735.22$^{ab}$ | 307.75 ± 508.08$^{ac}$ | 327.56 ± 667.51$^{acd}$ |
| Stage two | 322.9 ± 413.5$^{a}$ | 385.00 ± 304.9$^{ab}$ | 187.1 ± 203,6$^{abc}$ | 224.4 ± 319.2$^{abcd}$ |
| Stage three | 399.30 ± 560.40$^{a}$ | | | 335.50 ± 334.00$^{ab}$ |
| (Note: In the table, same letters in a row means that the difference of their values is insignificant, i.e. p>0.05, different letters in a same row means that the difference of their values is significant p<0.05; different letter size in a column means their values are significant, i.e. p<0.01) | | | | |

Table 24 above shows that, in the test Group I of cows, the number of somatic cells in milk decreased significantly. Similar decrease is observed in the milk produced by the test Group II of cows. The number of somatic cells in milk produced by control group of cows however increased. Since the number of somatic cells in milk is generally indicative of the overall health of the animals, the above data illustrates that the overall health of dairy animals administered with cysteamine will improve.

The above experimental results illustrate that a cysteamine composition according to the present invention is

effective for improving lactation of lactating animals.

## Claims

1. The use of a lactation increasing composition for improving lactation of lactating animals, wherein said composition comprises (i) 1 to 95wt% cysteamine or a salt thereof, (ii) an inclusion compound host material stabilizer for protecting said cysteamine or a salt thereof in said composition from light, heat, air and moisture of the surroundings, and (iii) a coated carrier, and wherein the use is non-therapeutic.

2. The use according to Claim 1 for the manufacture of a lactation improving feed or feed material.

3. The use of a composition containing cysteamine or a salt thereof for the manufacture of lactation improving medicament for lactating animals, wherein said composition comprises an inclusion compound host material stabilizer for protecting said cysteamine or a salt thereof from light, heat, air and moisture of the surroundings in said composition, and further comprises a coated carrier.

4. The use according to Claim 1, 2 or 3, wherein said improving lactation is an increase in milk yields.

5. The use according to any one of Claims 1 to 4, wherein said improving lactation is an increase in fat-corrected milk yields.

6. The use according to any one of Claims 1 to 5, wherein said improving lactation is an increase in milk fat content therein.

7. The use according to any one of Claims 1 to 6, wherein said improving lactation is an increase in milk protein content therein.

8. The use according to any one the preceding claims wherein said composition comprises 1 to 80wt% of said stabilizer, preferably 10wt% of said stabilizer.

9. The use according to any one of the preceding claims, wherein said stabilizer is selected from the group consisting of cyclodextrin, a derivative thereof and a combination thereof.

10. The use according to any one of the preceding claims,
wherein said cysteamine has a chemical formula of $NH_2$-$CH_2$-$CH_2$-SH or a salt thereof, preferably wherein said composition comprises 30wt% cysteamine.

11. The use according to Claim 3 or any one of Claims 4 to 7 as dependent on Claim 3, wherein said composition comprises 1 to 95wt% of said cysteamine or a salt thereof.

12. The use according to Claim 11, wherein said cysteamine has a chemical formula of $NH_2$-$CH_2$-$CH_2$-SH or a salt thereof, preferably wherein said composition comprises 30wt% cysteamine.

13. The use according to any one of claims 1 to 3, wherein the coated carrier is a solid carrier.

14. The use according to any one of claims 1 to 3, wherein said composition further comprises a bulking agent and/or a disintegration agent.

15. The use according to any one of claims 3, 13 or 14 wherein said carrier has a coating which is soluble in intestines of said animals.

16. The use according to any one of claims 3 or 13 to 15,
wherein said coated carrier exhibits a multi-layer structure in said composition.

17. The use according to any one of claims 3 or 13 to 16, wherein said coated carrier is adapted to remain un-dissolved at pH 1.5 to 3.5.

**18.** The use according to any one of the preceding claims, wherein said lactating animals are dairy cows.

**19.** The use according to Claim 2 or any one of Claims 4 to 13. as dependent on Claim 2, wherein said feed comprises 400 to 1000ppm of said composition, or in its dry state comprises 1000 to 2174ppm of said composition.

**20.** The use according to Claim 2 or any one of Claims 4 to 13 as dependent on Claim 2, wherein said feed comprises 120 to 300ppm of said cysteamine or a salt thereof, or in its dry state comprises 200 to 650ppm of said cysteamine or a salt thereof.

**21.** The use according to any one of Claims 2 or any one of Claims 4 to 13 as dependent on Claim 2 wherein said feed comprises other foodstuffs selected from a group including normal premix, cornmeal, cotton seed, wheat gluten, maize silage rutabaga, sugar beet pulp, apple pulp, ryegrass, fescue grass, alfalfa, feed concentrate and feed supplement.

**22.** A method of improving lactation of lactating animals comprising:

(a) producing a final feed by mixing a composition containing (i) cysteamine or a salt thereof, (ii) an inclusion compound host material stabilizer for protecting said cysteamine or salt thereof from light, heat, air and moisture of the surrounding, and (iii) a coated carrier with a suitable basal feed for said animals; and
(b) feeding said animals with said final feed;

wherein said method is non-therapeutic.

**23.** A method according to Claim 22, wherein said mixing in said step (a) comprises directly mixing said composition with said basal feed.

**24.** A method according to Claim 22, wherein said mixing in said step (a) comprises firstly preparing a premix including said cysteamine or a salt thereof, or said composition, and subsequently mixing said pre-mix with said basal feed.

**25.** A method according to Claim 24, wherein said premix is prepared by mixing said cysteamine or a salt thereof, or said composition, with a food material selected from a group including cornmeal.

**26.** A method according to Claim 24 or 25, wherein said premix comprises 5 to 25wt% of said composition.

**27.** A method according to Claim 24, 25 and 26, wherein said premix comprises 10 to 20wt% of said composition.

**28.** A method according to any one of Claims 22 to 29, comprising feeding said animals with 5.64 to 12.71g said cysteamine or a salt thereof, or 18.79 to 42.36g of said composition, per animal per day.

**29.** A method according to Claims 22 to 28, wherein said feed material comprises other foodstuffs selected from a group including normal premix, cornmeal, cotton seed, wheat gluten, maize silage rutabaga, sugar beet pulp, apple pulp, ryegrass, fescue grass, alfalfa, feed concentrate and/or feed supplement.

**30.** A method according to any one of Claims 22 to 29, wherein said feed material comprises 120 to 300ppm of said cysteamine, a salt or a combination thereof, or preferably 200 to 650ppm of said cysteamine, a salt or a combination thereof wherein in its dry state.

**31.** A method according to any one of Claims 22 to 29, wherein said feed material comprises from 400 to 1000ppm of said composition, or 1000 to 2174ppm wherein its dry state.

**Patentansprüche**

**1.** Verwendung einer laktationsfördernden Mischung zur Verbesserung der Laktation von laktierenden Tieren, wobei die Mischung umfasst,

(i) 1 bis 95 Gew.-% Cysteamin oder ein Salz davon,

(ii) einen Wirtsmaterial-Stabilisator für Einschlussverbindungen zum Schutz des Cysteamin oder eines Salzes davon in der Mischung vor Licht, Wärme, Luft und Feuchtigkeit aus der Umgebung und
(iii) einen beschichteten Trägerstoff

und
wobei die Verwendung nicht therapeutisch ist.

**2.** Verwendung nach Anspruch 1 zur Herstellung eines laktationsfördernden Futters oder Futtermaterials.

**3.** Verwendung einer Mischung, enthaltend Cysteamin oder ein Salz davon, zur Herstellung laktationsfördernder Arzneimittel für laktierende Tiere, wobei die Mischung einen Wirtsmaterial-Stabilisator für Einschlussverbindungen zum Schutz des Cysteamin oder eines Salzes davon in der Mischung vor Licht, Wärme, Luft and Feuchtigkeit aus der Umgebung, sowie ferner einen beschichteten Trägerstoff umfasst.

**4.** Verwendung nach einem der Ansprüche 1, 2 oder 3, wobei die sich verbessernde Laktation eine Zunahme der Milchausbeuten darstellt.

**5.** Verwendung nach einem der Ansprüche 1 bis 4, wobei die sich verbessernde Laktation eine Zunahme der Ausbeuten an fettkorrigierter Milch darstellt.

**6.** Verwendung nach einem der Ansprüche 1 bis 5, wobei die sich verbessernde Laktation eine Zunahme an Milchfettgehalt dabei darstellt.

**7.** Verwendung nach einem der Ansprüche 1 bis 6, wobei die sich verbessernde Laktation eine Zunahme an Milchproteingehalt dabei darstellt.

**8.** Verwendung nach einem der vorhergehenden Ansprüche, wobei die Mischung 1 bis 80 Gew.-% des Stabilisators, vorzugsweise 10 Gew.-% des Stabilisators, umfasst.

**9.** Verwendung nach einem der vorhergehenden Ansprüche, wobei der Stabilisator ausgewählt ist aus der Gruppe bestehend aus Cyclodextrin, ein Derivat davon und eine Kombination davon.

**10.** Verwendung nach einem der vorhergehenden Ansprüche, wobei das Cysteamin die chemische Formel $NH_2$-$CH_2$-$CH_2$-SH hat oder ein Salz davon ist,
wobei die Mischung vorzugsweise 30 Gew.-% an Cysteamin umfasst.

**11.** Verwendung nach Anspruch 3 oder einem der Ansprüche 4 bis 7, der auf Anspruch 3 rückbezogen ist, wobei die Mischung 1 bis 95 Gew.-% des Cysteamin oder eines Salzes davon umfasst.

**12.** Verwendung nach Anspruch 11, wobei das Cysteamin die chemische Formel $NH_2$-$CH_2$-$CH_2$-SH hat oder ein Salz davon ist, wobei die Mischung vorzugsweise 30 Gew.-% an Cysteamin umfasst.

**13.** Verwendung nach einem Ansprüche 1 bis 3, wobei der beschichtete Trägerstoff ein fester Trägerstoff ist.

**14.** Verwendung nach einem Ansprüche 1 bis 3, wobei die Mischung ferner einen Füllstoff und/oder ein Desintegrationsmittel umfasst.

**15.** Verwendung nach einem Ansprüche 3, 13 oder 14, wobei der Trägerstoff eine Beschichtung hat, die im Intestinalbereich der Tiere löslich ist.

**16.** Verwendung nach einem Ansprüche 3 oder 13 bis 15, wobei der Trägerstoff in der Mischung einen mehrschichtigen Aufbau aufweist.

**17.** Verwendung nach einem Ansprüche 3 oder 13 bis 16, wobei der Trägerstoff geeignet ist bei einem pH von 1,5 bis 3,5 ungelöst zu bleiben.

**18.** Verwendung nach einem der vorhergehenden Ansprüche, wobei die laktierenden Tiere Milchkühe sind.

**19.** Verwendung nach Anspruch 2 oder einem der Ansprüche 4 bis 13, der auf Anspruch 2 rückbezogen ist, wobei das Futter 400 bis 1000 ppm der Mischung, oder im Trockenzustand 1000 bis 2174 ppm der Mischung umfasst.

**20.** Verwendung nach Anspruch 2 oder einem der Ansprüche 4 bis 13, der auf Anspruch 2 rückbezogen ist, wobei das Futter 120 bis 300 ppm des Cysteamin oder eines Salzes davon, oder im Trockenzustand 200 bis 650 ppm des Cysteamin oder eines Salzes davon umfasst.

**21.** Verwendung nach Anspruch 2 oder einem der Ansprüche 4 bis 13, der auf Anspruch 2 rückbezogen ist, wobei das Futter weitere Nahrungsmittel, ausgewählt aus der Gruppe bestehend aus normale Vormischung, Maismehl, Baumwollsamen, Gluten, Mais-Rüben-Silage, Zuckerrübenpulp, Apfelpulp, Raigras, Schwingel, Alfalfa, Futterkonzentrat und Nahrungsmittelzusatz, umfasst.

**22.** Verfahren zur Verbesserung der Laktation von laktierenden Tieren, umfassend die Schritte:

a) Produzieren eines fertigen Futters durch Mischen einer Mischung, enthaltend (i) Cysteamin oder ein Salz davon, (ii) einen Wirtsmaterial-Stabilisator für Einschlussverbindungen zum Schutz des Cysteamin oder Salzes davon vor Licht, Wärme, Luft and Feuchtigkeit aus der Umgebung, und (iii) einen beschichteten Trägerstoff, mit einem für die Tiere geeigneten Basisfutter und
b) Füttern der Tiere mit dem Fertigfutter,

wobei das Verfahren nicht therapeutisch ist.

**23.** Verfahren nach Anspruch 22, wobei das Mischen in Schritt a) ein direktes Mischen der Mischung mit dem Basisfutter umfasst.

**24.** Verfahren nach Anspruch 22, wobei das Mischen in Schritt a) zunächst das Bereitstellen einer Vormischung, enthaltend das Cysteamin oder ein Salz davon oder die Mischung, und anschließend das Mischen der Vormischung mit dem Basisfutter umfasst.

**25.** Verfahren nach Anspruch 24, wobei die Vormischung bereitgestellt wird indem das Cysteamin oder ein Salz davon oder die Mischung mit einem Nahrungsmittelmaterial, ausgewählt aus einer Gruppe umfassend Maismehl, vermischt wird.

**26.** Verfahren nach Anspruch 24 oder 25, wobei die Vormischung 5 bis 25 Gew.-% der Mischung umfasst.

**27.** Verfahren nach Anspruch 24, 25 und 26, wobei die Vormischung 10 bis 20 Gew.-% der Mischung umfasst.

**28.** Verfahren nach einem der Ansprüche 22 bis 27, umfassend das Füttern der Tiere mit 5,64 bis 12,71 g des Cysteamin oder eines Salzes davon, oder mit 18,79 bis 42,36 g der Mischung, pro Tier pro Tag.

**29.** Verfahren nach einem der Ansprüche 22 bis 28, wobei das Futtermaterial weitere Nahrungsmittel, ausgewählt aus einer Gruppe umfassend normale Vormischung, Maismehl, Baumwollsamen, Gluten, Mais-Rüben-Silage, Zuckerrübenpulp, Apfelpulp, Raigras, Schwingel, Alfalfa, Futterkonzentrat, und/oder Futterzusatz, umfasst.

**30.** Verfahren nach einem der Ansprüche 22 bis 29, wobei das Futtermaterial 120 bis 300 ppm des Cysteamin, eines Salzes oder einer Kombination davon oder vorzugsweise 200 bis 650 ppm des Cysteamin, eines Salzes oder einer Kombination davon im Trockenzustand umfasst.

**31.** Verfahren nach einem der Ansprüche 22 bis 29, wobei das Futtermaterial im Wesentlichen 400 bis 1000 ppm der Mischung oder 1000 bis 2174 ppm in ihrem Trockenzustand, umfasst.

### Revendications

**1.** Utilisation d'une composition de stimulation de la lactation pour améliorer la lactation d'animaux en lactation, dans laquelle ladite composition comprend (i) 1 à 95 % en poids de cystéamine ou un sel de celle-ci, (ii) un stabilisant de matériau hôte de composé d'inclusion pour protéger ladite cystéamine ou un sel de celle-ci dans ladite composition de la lumière, de la chaleur, de l'air et de l'humidité de l'environnement, et (iii) un support revêtu, et

dans laquelle l'utilisation est non thérapeutique.

2. Utilisation selon la revendication 1 pour la fabrication d'une alimentation ou d'un matériau d'alimentation améliorant la lactation.

3. Utilisation d'une composition contenant de la cystéamine ou un sel de celle-ci pour la fabrication d'un médicament améliorant la lactation pour des animaux en lactation, dans laquelle ladite composition comprend un stabilisant de matériau hôte de composé d'inclusion pour protéger ladite cystéamine ou un sel de celle-ci de la lumière, de la chaleur, de l'air et de l'humidité de l'environnement dans ladite composition, et comprend en outre un support revêtu.

4. Utilisation selon la revendication 1, 2 ou 3, dans laquelle ladite amélioration de lactation est une augmentation de la production de lait.

5. Utilisation selon l'une quelconque des revendications 1 à 4, dans laquelle ladite amélioration de lactation est une augmentation de la production de lait standard.

6. Utilisation selon l'une quelconque des revendications 1 à 5, dans laquelle ladite amélioration de lactation est une augmentation de la teneur en matières grasses du lait dans celui-ci.

7. Utilisation selon l'une quelconque des revendications 1 à 6, dans laquelle ladite amélioration de lactation est une augmentation de la teneur en protéines du lait dans celui-ci.

8. Utilisation selon l'une quelconque des revendications précédentes, dans laquelle ladite composition comprend 1 à 80 % en poids dudit stabilisant, de préférence 10 % en poids dudit stabilisant.

9. Utilisation selon l'une quelconque des revendications précédentes, dans laquelle ledit stabilisant est choisi dans le groupe constitué par la cyclodextrine, un dérivé de celle-ci et une combinaison de celle-ci.

10. Utilisation selon l'une quelconque des revendications précédentes, dans laquelle ladite cystéamine a une formule chimique de $NH_2$-$CH_2$-$CH_2$-SH ou un sel de celle-ci, de préférence dans laquelle ladite composition comprend 30 % en poids de cystéamine.

11. Utilisation selon la revendication 3 ou l'une quelconque des revendications 4 à 7 dépendantes de la revendication 3, dans laquelle ladite composition comprend 1 à 95 % en poids de ladite cystéamine ou d'un sel de celle-ci.

12. Utilisation selon la revendication 11, dans laquelle ladite cystéamine a une formule chimique de $NH_2$-$CH_2$-$CH_2$-SH ou un sel de celle-ci, de préférence dans laquelle ladite composition comprend 30 % en poids de cystéamine.

13. Utilisation selon l'une quelconque des revendications 1 à 3, dans laquelle le support revêtu est un support solide.

14. Utilisation selon l'une quelconque des revendications 1 à 3, dans laquelle ladite composition comprend en outre un diluant et/ou un agent de désintégration.

15. Utilisation selon l'une quelconque des revendications 3, 13 ou 14, dans laquelle ledit support présente un revêtement soluble dans les intestins desdits animaux.

16. Utilisation selon l'une quelconque des revendications 3 ou 13 à 15, dans laquelle ledit support revêtu présente une structure multicouche dans ladite composition.

17. Utilisation selon l'une quelconque des revendications 3 ou 13 à 16, dans laquelle ledit support revêtu est adapté pour rester non dissous à un pH de 1,5 à 3,5.

18. Utilisation selon l'une quelconque des revendications précédentes, dans laquelle lesdits animaux en lactation sont des vaches laitières.

19. Utilisation selon la revendication 2 ou l'une quelconque des revendications 4 à 13 dépendantes de la revendication 2, dans laquelle ladite alimentation comprend 400 à 1 000 ppm de ladite composition, ou dans son état sec comprend

1 000 à 2 174 ppm de ladite composition.

**20.** Utilisation selon la revendication 2 ou l'une quelconque des revendications 4 à 13 dépendantes de la revendication 2, dans laquelle ladite alimentation comprend 120 à 300 ppm de ladite cystéamine ou d'un dit sel de celle-ci, ou dans son état sec comprend 200 à 650 ppm de ladite cystéamine
ou d'un sel de celle-ci.

**21.** Utilisation selon l'une quelconque de la revendication 2 ou l'une quelconque des revendications 4 à 13 dépendantes de la revendication 2, dans laquelle ladite alimentation comprend d'autres aliments choisis dans un groupe comprenant le prémélange normal, de la semoule de maïs, des graines de coton, du gluten de blé, du rutabaga d'ensilage de maïs-fourrage, de la pulpe de betterave, de la pulpe de pomme, du fromental, du seigle bâtard, de la luzerne, des aliments concentrés pour animaux et des suppléments protéiques.

**22.** Procédé d'amélioration de la lactation d'animaux en lactation comprenant les étapes consistant à :

(a) produire une alimentation finale en mélangeant une composition contenant (i) de la cystéamine ou un sel de celle-ci, (ii) un stabilisant de matériau hôte de composé d'inclusion pour protéger ladite cystéamine ou ledit sel de celle-ci, de la lumière, de la chaleur, de l'air et de l'humidité de l'environnement et (iii) un support revêtu avec une alimentation de base appropriée pour lesdits animaux ; et
(b) nourrir lesdits animaux avec ladite alimentation finale ;

dans lequel ledit procédé est non thérapeutique.

**23.** Procédé selon la revendication 22, dans lequel ledit mélange dans ladite étape (a) comprend le mélange direct de ladite composition avec ladite alimentation de base.

**24.** Procédé selon la revendication 22, dans lequel ledit mélange dans ladite étape (a) comprend tout d'abord la préparation d'un prémélange comprenant ladite cystéamine ou un sel de celle-ci ou ladite composition, puis le mélange dudit prémélange avec ladite alimentation de base.

**25.** Procédé selon la revendication 24, dans lequel ledit prémélange est préparé par mélange de ladite cystéamine ou d'un sel de celle-ci ou de ladite composition avec un matériau alimentaire choisi dans un groupe comprenant la semoule de maïs.

**26.** Procédé selon la revendication 24 ou 25, dans lequel ledit prémélange comprend 5 à 25 % en poids de ladite composition.

**27.** Procédé selon les revendications 24, 25 et 26, dans lequel ledit prémélange comprend 10 à 20 % en poids de ladite composition.

**28.** Procédé selon l'une quelconque des revendications 22 à 29, comprenant l'alimentation desdits animaux avec 5,64 à 12,71 g de ladite cystéamine ou d'un sel de celle-ci ou 18,79 à 42,36 g de ladite composition par animal et par jour.

**29.** Procédé selon les revendications 22 à 28, dans lequel ledit matériau d'alimentation comprend d'autres aliments choisis dans un groupe comprenant le prémélange normal, de la semoule de maïs, des graines de coton, du gluten de blé, du rutabaga d'ensilage de maïs-fourrage, de la pulpe de betterave, de la pulpe de pomme, du fromental, du seigle bâtard, de la luzerne, des aliments concentrés pour animaux et/ou des suppléments protéiques.

**30.** Procédé selon l'une quelconque des revendications 22 à 29, dans lequel ledit matériau d'alimentation comprend 120 à 300 ppm de ladite cystéamine, d'un sel ou d'une combinaison de ceux-ci, ou de préférence 200 à 650 ppm de ladite cystéamine, d'un sel ou d'une combinaison de ceux-ci lorsqu'il se trouve à l'état sec.

**31.** Procédé selon l'une quelconque des revendications 22 à 29, dans lequel ledit matériau d'alimentation comprend 400 à 1 000 ppm de ladite composition ou 1 000 à 2 174 ppm lorsqu'il se trouve à l'état sec.

FIG.1

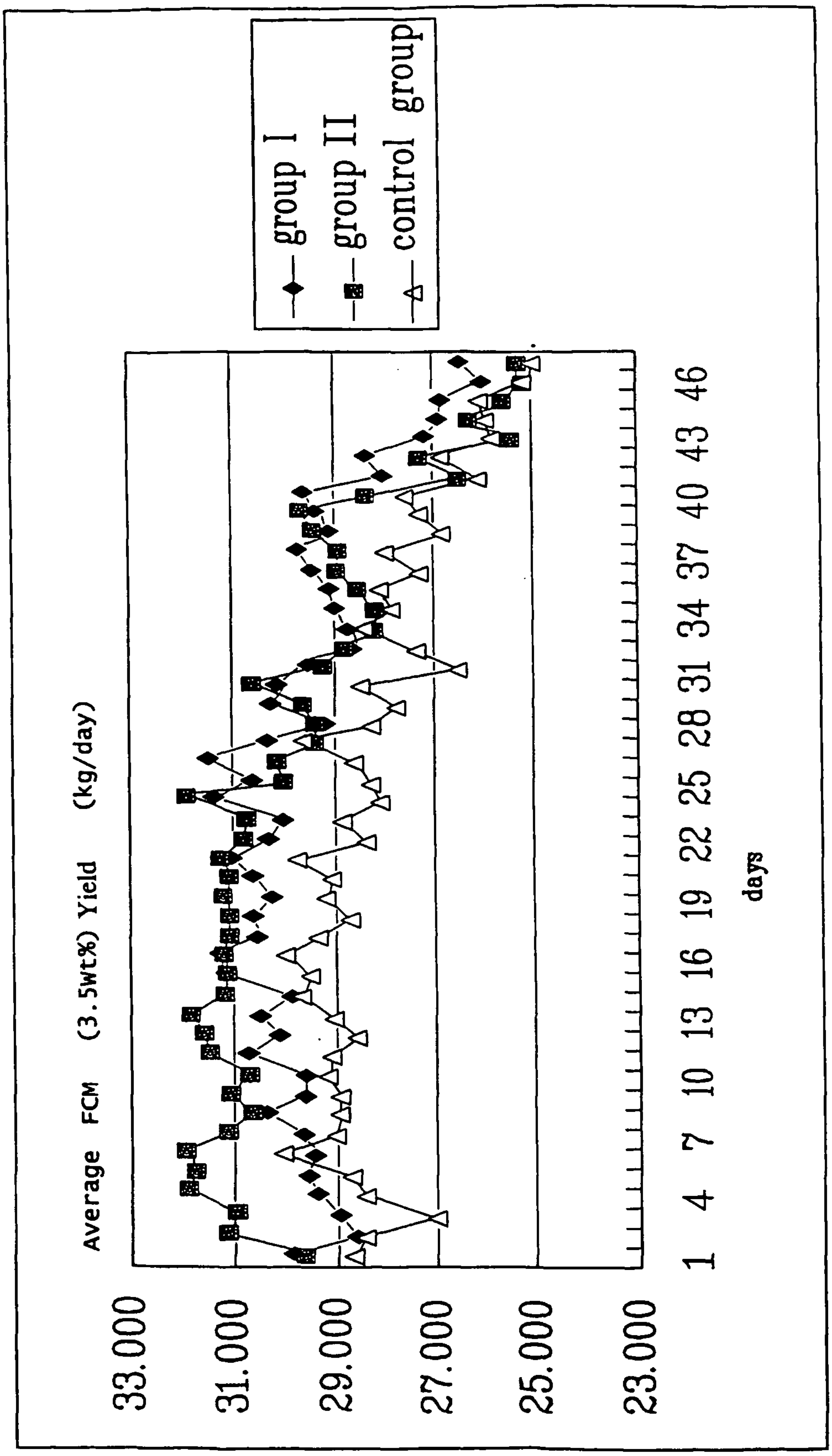
Average FCM (3.5wt%) Yield (kg/day)
33.000
31.000
29.000
27.000
25.000
23.000
1 4 7 10 13 16 19 22 25 28 31 34 37 40 43 46
days
group I
group II
control group

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4711897 A
- CN 1358499
- WO 0248110 A


### Non-patent literature cited in the description


- **VON F. ROTT.** *Tierärztl. Umschau,* 1988, vol. 43, 324-328
- **WANG.** *Zoological Research,* 1998, vol. 19 (3), 247-249